# EUROPÄISCHE PATENTSCHRIFT

(11) **EP 1 833 786 B1**
(45) Veröffentlichungstag und Bekanntmachung des Hinweises auf die Patenterteilung: **17.02.2010**
(21) Anmeldenummer: 05822049.2
(22) Anmeldetag: 22.12.2005
(51) Int. Cl.: C07C 381/00, A62D 1/00, B01F 17/00

(54) **FLUORTENSIDE**
FLUOROSURFACTANTS
AGENTS TENSIOACTIFS FLUORES

(30) Priorität: 05.01.2005 DE 102005000858
(43) Veröffentlichungstag der Anmeldung: 19.09.2007
(73) Patentinhaber: Merck Patent GmbH, 64293 Darmstadt (DE)
(72) Erfinder: KIRSCH, Peer, Kanagawa 224-0033 (JP); FRANZ, Klaus-Dieter, 65779 Kelkheim (DE); RUHL, Andreas, 64380 Rossdorf (DE)
(86) Internationale Anmeldenummer: PCT/EP2005/013859
(87) Internationale Veröffentlichungsnummer: WO 2006/072401

(56) Entgegenhaltungen:
- US-A- 3 102 903
- US-A- 4 849 540
- US-A- 6 110 976
- US-A1- 2003 138 380

## Beschreibung

Gegenstand der vorliegenden Erfindung ist die Verwendung von Verbindungen enthaltend Endgruppen Y, wobei Y steht für CF₃O- oder F₅S- als oberflächenaktiven Verbindungen, entsprechende neue Verbindungen und Herstellverfahren für diese Verbindungen.

Fluortenside besitzen eine überragende Fähigkeit zur Senkung der Oberflächenenergie, die beispielsweise bei der Hydrophobisierung von Oberflächen, wie der Textilimprägnierung, der Hydrophobisierung von Glas, oder dem sogenannten Enteisen von Flugzeugtragflächen, genutzt wird.

In der Regel enthalten Fluortenside jedoch Perfluoralkylsubstituenten, die in der Umwelt durch biologische und andere Oxidationsprozesse zu Perfluoralkancarbonsäuren und -sulfonsäuren abgebaut werden. Diese gelten als persistent und stehen z. T. im Verdacht gesundheitliche Schäden zu verursachen (G. L. Kennedy, Jr., J. L. Butenhoff, G. W. Olsen, J. C. O'Connor, A. M. Seacat, R. G. Perkins, L. B. Biegel, S. R. Murphy, D. G. Farrar, Critical Reviews in Toxicology 2004, 34, 351-384). Längerkettige Perfluoratkancarbonsäuren und -sulfonsäuren reichem sich zudem in der Nahrungskette an.

Daher besteht Bedarf nach oberflächenaktive Substanzen mit einem den klassischen Fluortensiden vergleichbaren Eigenschaftsprofil, die beim oxidativen oder reduktiven Abbau jedoch keine persistenten fluororganischen Abbauprodukte hinterlassen.

Von der Firma Omnova werden Polymere vertrieben, deren Seitenketten terminale CF₃- oder C₂F₅-Gruppen aufweisen. In der Internationalen Patentanmeldung WO 03/010128 werden Perfluoralkyl-substituierte Amine, Säuren, Aminosäuren und Thioethersäuren beschrieben, die eine C₃₋₂₀-Perfluoralkyl-Gruppe aufweisen.

Aus JP-A-2001/133984 sind oberflächenaktive Verbindungen mit Perfluoralkoxy-Ketten bekannt, die sich zum Einsatz in Antireflex-Beschichtungen eignen. Aus JP-A-09/111286 ist die Verwendung von Pefluorpolyethertensiden in Emulsionen bekannt. Aus US 6,110,976 sind fluorierte cyclohexanderivative bekannt, die perfluorierte Alkylgruppen tragen.

US 2003/0138380 beschreibt Gasemulsionen enthaltend Mono- und Polyether, die ggf. CF₃O-Gruppen enthalten können.

Alle bisher bekannten Fluortenside führen aber beim Abbau letztendlich zur Bildung persistenter Perfluoralkansulfonsäuren und -carbonsäuren. Selbst die als ökologisch besser verträglich eingeführten Ersatzstoffe mit einer terminalen CF₃-Gruppe können zu persistenter Trifluoressigsäure abgebaut werden. Daher besteht weiterhin Bedarf nach weiteren, vollständig abbaubaren Substituten für perfluorierte Tenside.

Jetzt wurde gefunden, dass sich Verbindungen, die mindestens eine endständige Pentafluorsulfuranyl-Gruppe oder mindestens eine endständige Trifluormethoxy-Gruppe tragen und über eine polare Endgruppe verfügen, oberflächenaktiv sind und sich in hervorragender Weise als Tenside eignen.

Ein erster Gegenstand der vorliegenden Erfindung ist daher die Verwendung gemäß Anspruch 1.

Dabei ist die Endgruppe Y in den oberflächenaktiven Verbindungen an eine gesättigte oder ungesättigte, verzweigte oder unverzweigte Kohlenwasserstoff-Einheit gebunden. Bei den Kohlenwasserstoff-Einheiten kann es sich um aliphatische oder aromatische, gegebenenfalls mit Heteroatomen versehene Einheiten handeln.

Dabei enthalten die erfindungsgemäß zu verwendenden Verbindungen neben den genannten fluorierten Endgruppen vorzugsweise keine weiteren fluorierten Gruppen.

In einer Erfindungsvariante kommt die Endgruppe Y in der oberflächenaktiven Verbindung mehrfach vor und es handelt sich bei der oberflächenaktiven Verbindung vorzugsweise um ein Oligomer oder Polymer.

In einer anderen ebenfalls bevorzugten Erfindungsvariante kommt die Endgruppe Y in der oberflächenaktiven Verbindung nur einmal, zweimal oder dreimal vor, wobei Verbindungen in denen die Endgruppe nur einmal vorkommt insbesondere bevorzugt sind. Dabei handelt es sich bei den erfindungsgemäß zu verwendenden Verbindungen vorzugsweise um niedermolekulare Verbindungen der Formel I

Y-Spacer-X I,

wobei
- Y steht für CF₃O- oder F₅S-,
- Spacer steht für eine gesättigte oder ungesättigte, verzweigte oder unverzweigte Kohlenwasserstoff-Einheit,
- X steht für eine kationische, nichtionische, ampothere oder anionische polare Gruppe oder eine polymerisierbare Gruppe.

Die Verbindung der Formel I ist ausgewahlt aus den Verbindungen der Formel Ia, Ib oder Ic,

Y-(CH₂)n-X Ia

Y-CH₂-CH(Hal)-(CH₂) ₍ₙ₋₁₎-X Ib

Y-CH=CH-(CH₂)₍ₙ₋₁₎-X Ic

worin Y steht für CF₃O- oder F₅S-,
n steht für eine ganze Zahl aus dem Bereich 4 bis 28 und
X steht für eine kationische, nichtionische, ampothere oder anionische polare Gruppe oder eine polymerisierbare Gruppe,
(Hal) steht für F, Cl, Br oder I,
sowie entsprechender Salze der Verbindungen nach Formel Ia, Ib bzw. Ic.

Ganz besonders bevorzugt ist dabei die Verwendung von Verbindungen der Formel Ia, wobei n insbesondere bevorzugt steht für eine ganze Zahl aus dem Bereich von 4 bis 24, und besonders bevorzugt für eine ganze Zahl aus dem Bereich 6 bis 18. Dabei ist es in einer Erfindungsvariante wiederum bevorzugt, wenn n geradzahlig ist.

Erfindungsgemäß insbesondere bevorzugt ist die Verwendung der oben genannten Verbindungen als Tensid.

Handelt es sich bei den Verbindungen nach Formel I um anionische Verbindungen oder anionisch versalzbare Verbindungen, so ist es bevorzugt, wenn als Gegenion ein Alkalimetall-Ion, vorzugsweise Li⁺, Na⁺ oder K⁺, ein Erdalkalimetall-Ion oder NH₄⁺ vorliegt. Handelt es sich bei den Verbindungen nach Formel I um kationische Verbindungen oder kationisch versalzbare Verbindungen, so ist es bevorzugt, wenn als Gegenion ein Halogenid, wie Cl⁻, Br⁻, I⁻, oder CH₃SO₃⁻, CF₃SO₃⁻, CH₃PhSO₃⁻ oder PhSO₃⁻ vorliegt.

Aus der Literatur sind entsprechende bzw. strukturell ähnliche Verbindungen teilweise bekannt:
- In der Beilstein-Datenbank (Beilstein Institut zur Förderung der chemischen Wissenschaften; 2003) sind u.a. die Verbindungen 2-(Pentafluorsulfuranyl)ethanol, Carboethoxymethylschwefel-pentafluorid, Pentafluorsulfuranylessigsäure und Pentafluorsulfuranylethansulfonsäure beschrieben.
- Die Herstellung von Pentafluorsulfuranylacetaldehyd und Pentafluorsulfuranylessigsäure wird in N. H. Ray, J. Chem. Soc., Abstracts 1963, 1440 bzw. GB 941,392 und GB 941,393 beschrieben. In US 3,102,903 wird die Herstellung verschiedener Pentafluorsulfuranylessigsäure-Derivate beschrieben.
- Orthocarbonate und Carbamate des Pentafluorsulfuranylethanol mit Nitro-Endgruppen und deren Verwendung als Explosivstoff werden in US 4,849,540 beschrieben.
- Aus A.F.T. Yokochi, R. Winter, G.Gard, Acta Cryst. 2002, E58, o1133-o1135 ist die Kristallstruktur von 3-Pentafluorsulfuranylpropionsäure bekannt.
- Die Herstellung verschiedener 3-Pentafluorsulfuranylpropan-Derivate wird in R. Winter, G. L. Gard, J. Fluorine Chem. 2000, 102, 79-87 beschrieben. Durch Umsetzung von SF₅Br mit Acrylsäureestern und anschließende Modifikation der Estergruppe werden 3-Pentafluorsulfuranylpropionsäure, 3-Pentafluorsulfuranylpropanol und 3-Brom-1-pentafluorsulfuranylpropan erhalten.
- Pentafluorsulfuranylmethansulfonate SF₅CX₂SO₃⁻ mit X = H bzw. F werden in B. H. Ward, J. A. Schlueter, U. Geiser, H. H. Wang, E. Morales, J. P. Parakka, S. Y. Thomas, J. M. Williams, P. G. Nixon, R. W. Winter, G. L. Gard, H.-J. Koo, M.-H. Whangboo, Chem.Mater. 2000, 12, 343-351 beschrieben.
- Das Pentafluorsulfuranylethansulfonat wird in J. P. Canselier, J. L. Boyer, V. Castro, G. L. Gard, J. Mohtasam, D. H. Peyton, Magn. Reson. Chem. 1995, 33, 506-510 beschrieben.
- In R. Winter, G. I. Gard, J. Fluorine Chem. 1994, 66, 109-116 wird die Herstellung verschiedener Ester von Pentafluorsulfuranylethanolen und -propanolen beschrieben.
- In WO 2004/011422 wird die Herstellung aliphatischer und aromatischer Verbindungen mit Pentafluorsulfuranyl-Substituenten beschrieben. Die Herstellung erfolgt dabei durch Addition von SF₅Cl an Doppelbindungen. Hergestellt werden beispielsweise Verbindungen des Typs F₅S-CH₂-CHCl-(CH₂)₈-X¹ wobei X¹ steht für OH, OC(=O)CH₃, Br, C(=O)OC₂H₅ bzw. C(=O)CH₃,
Vorteile der erfindungsgemäßen Verbindungen bzw. erfindungsgemäßen Verwendung der genannten Verbindungen bzw. der erfindungsgemäßen Zusammensetzungen können dabei insbesondere sein:
- eine Oberflächenaktivität, die der konventioneller Kohlenwasserstoff-Tenside hinsichtlich Effizienz und/oder Effektivität überlegen ist,
- biologische und/oder abiotische Abbaubarkeit der Substanzen ohne Bildung persistenter, perfluorierter Abbauprodukte,
- gute Verarbeitbarkeit in Formulierungen,
- Lagerstabilität.

Ein weiterer Gegenstand der vorliegenden Erfindung sind dabei die entsprechenden neuen Verbindungen nach Formel IIa, IIb bzw. IIc

CF₃O-(CH₂)ₙ-X IIa

CF₃O-CH₂-CH(Hal)-(CH₂)₍ₙ₋₁₎-X IIb

CF₃O-CH=CH-(CH₂)₍ₙ₋₁₎-X IIc

worin
n steht für eine ganze Zahl aus dem Bereich 4 bis 28 und
X steht für eine kationische, nichtionische, ampothere oder anionische polare Gruppe oder eine polymerisierbare Gruppe, (Hal) steht für F, Cl, Br oder I,
sowie entsprechende Salze der Verbindungen nach Formel IIa, IIb bzw. IIc, wobei CF₃-O-CH₂-COOH ausgenommen ist,
bzw. Verbindung nach Formel IIIa, IIIb oder IIIc

F₅S- (CH₂)ₙ-X IIIa

F₅S-CH₂-CH(Hal)-(CH₂)₍ₙ₋₁₎-X IIIb

F₅S-CH=CH-(CH₂)₍ₙ₋₁₎-X IIIc

worin
n steht für eine ganze Zahl aus dem Bereich 4 bis 28 und
X steht für eine kationische, nichtionische, ampothere oder anionische polare Gruppe oder eine polymerisierbare Gruppe,
(Hal) steht für F, Cl, Br oder I,
sowie entsprechende Salze der Verbindungen nach Formel IIIa, IIIb bzw. IIIc, wobei die Verbindungen F₅S-(CH₂)₁-CO₂M, F₅S-(CH₂)₂-CO₂M, F₅S-(CH₂)₁-SO₃M, F₅S-(CH₂)₂-SO₃M, F₅S-(CH₂)₁-CONH₂, F₅S-(CH₂)₂-OH und F₅S-(CH₂)₃-OH, F₅S-CH₂-CHCl-(CH₂)₈-X¹ wobei M steht für H oder ein Alkalimetall-Ion, vorzugsweise Li⁺, Na⁺ oder K⁺, oder NH₄⁺ und X¹ steht für OH, OC(=O)CH₃, Br, C(=O)OC₂H₅, C(=O)CH₃, ausgenommen sind.

Vorzugsweise steht n bei Verbindungen der Formeln I bzw. II bzw. III für eine Zahl aus dem Bereich 8 bis 24.

In einer bevorzugten Gruppe von erfindungsgemäß einzusetzenden Verbindungen nach Formel I bzw. erfindungsgemäßen Verbindungen nach den Formeln II oder III steht X für eine anionische polare Gruppe ausgewählt aus -COOM, -SO₃M, -OSO₃M, -PO₃M₂, -OPO₃M₂, - (OCH₂CH₂)ₘ-O-(CH₂)ₒ-COOM, -(OCH₂CH₂)ₘ-O-(CH₂)ₒ-SO₃M, - (OCH₂CH₂)ₘ-O-(CH₂)ₒ-OSO₃M, -(OCH₂CH₂)ₘ-O-(CH₂)ₒ-PO₃M₂, -(OCH₂CH₂)ₘ-O-(CH₂)ₒ-OPO₃M₂, wobei M steht für H oder ein Alkalimetall-Ion, vorzugsweise Li⁺, Na⁺ oder K⁺, oder NH₄⁺, m steht für eine ganze Zahl aus dem Bereich von 1 bis 1000 und o steht für eine ganze Zahl ausgewählt aus 1, 2, 3 oder 4.

Zu den bevorzugten anionischen Gruppen gehören dabei insbesondere-COOM, -SO₃M, -OSO₃M, sowie -(OCH₂CH₂)ₘ-O-(CH₂)ₒ-COOM, -(OCH₂CH₂)ₘ-O-(CH₂)ₒ-SO₃M und -(OCH₂CH₂)ₘ-O-(CH₂)ₒ-OSO₃M, wobei jede einzelne dieser Gruppen für sich genommen bevorzugt sein kann.

In einer anderen ebenfalls bevorzugten Gruppe von erfindungsgemäß einzusetzenden Verbindungen nach Formel I bzw. erfindungsgemäßen Verbindungen nach den Formeln II oder III steht X für eine kationische polare Gruppe ausgewählt aus -NR¹R²R³⁺ Z⁻, -PR¹R²R³⁺ Z⁻, 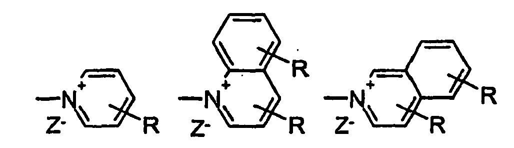 wobei R steht für H oder C₁₋₄-Alkyl in beliebiger Position,
Z⁻ steht für Cl⁻, Br⁻, I⁻, CH₃SO₃⁻, CF₃SO₃⁻, CH₃PhSO₃⁻, PhSO₃⁻ R¹, R² und R³ jeweils unabhängig voneinander stehen für H, C₁₋₃₀-Alkyl, Ar oder -CH₂Ar und
Ar steht für einen unsubstituierten oder ein- oder mehrfach substituierten aromatischen Ring oder kondensierte Ringsysteme mit 6 bis 18 C-Atomen, worin auch ein oder zwei CH-Gruppen durch N ersetzt sein können.

Zu den bevorzugten kationischen Gruppen gehören dabei insbesondere aus -NR¹R²R³⁺ Z und 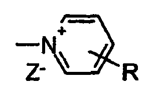 , wobei jede einzelne dieser Gruppen für sich genommen bevorzugt sein kann.

In einer weiteren bevorzugten Gruppe von erfindungsgemäß einzusetzenden Verbindungen nach Formel I bzw. erfindungsgemäßen Verbindungen nach den Formeln II oder III steht X für eine nicht-ionische polare Gruppe ausgewählt aus -Cl, -Br, -I, -(OCH₂CH₂)ₘ-OH, -O-(Glycosid)ₒ, -(OCH₂CH₂)ₘ-OCH₂-CHOH-CH₂-OH, -(OCH₂CH₂)ₘ-OCH₂Ar(-NCO)ₚ, -(OCH₂CH₂)ₘ-OAr(-NCO)ₚ, -SiR¹R²Z, -SiR¹Z₂, -SiZ₃, -COZ, -(OCH₂CH₂)ₘ-SO₂CH=CH₂, -SO₂Z, 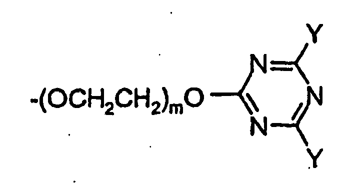
m steht für eine ganze Zahl aus dem Bereich von 0 bis 1000,
n steht für 0 oder 1 und
o steht für eine ganze Zahl aus dem Bereich von 1 bis 10,
p steht für 1 oder 2,
R¹ und R² jeweils unabhängig voneinander stehen für C₁₋₃₀-Alkyl, Ar oder -CH₂Ar und,
Ar steht für einen unsubstituierten, ein- oder mehrfach substituierten aromatischen Ring oder kondensierte Ringsysteme mit 6 bis 18 C-Atomen, worin auch ein oder zwei CH-Gruppen durch C=O ersetzt sein können, und
Glycosid steht für ein verethertes Kohlenhydrat, vorzugsweise für ein mono- di-, tri- oder oligo-Glucosid,
alle Z jeweils unabhängig voneinander stehen für -H, -Cl, -F, -NR¹R², -OR¹, -N-Imidazolyl und
Y steht für Cl oder F.

Zu den bevorzugten nicht-ionischen polaren Gruppen gehören dabei insbesondere -(OCH₂CH₂)ₘ-OH und -O-(Glycosid)ₒ, wobei jede einzelne dieser Gruppen für sich genommen bevorzugt sein kann.

Darüber hinaus können erfindungsgemäß solche Verbindungen der Formeln I, II oder III bevorzugt sein bzw. bevorzugt verwendet werden, bei denen X steht für eine polymerisierbare Gruppe ausgewählt aus-(OCH₂CH₂)ₘOCOCR=CH₂, -(OCH₂CH₂)ₘ-OCR=CH₂, 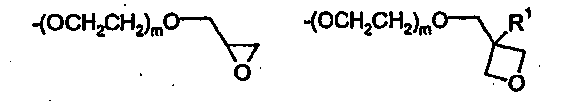 wobei m steht für eine ganze Zahl aus dem Bereich von 0 bis 1000 und R bzw. R¹ steht für H oder C₁₋₄-Alkyl. Diese Verbindungen werden vorzugsweise zu Polymeren mit entsprechenden Seitenketten verarbeitet, die selbst wieder im erfindungsgemäßen Sinne eingesetzt werden können. Auch die Verwendung dieser Polymere ist ein Gegenstand der vorliegenden Erfindung.

Darüber hinaus können erfindungsgemäß solche Verbindungen bevorzugt sein bzw. bevorzugt verwendet werden, bei denen X steht für eine amphotere Gruppe ausgewählt aus den funktionellen Gruppen der Acetyldiamine, der N.-Alkylaminosäuren, der Betaine, der Aminoxide bzw. entsprechender Derivate. In bevorzugten Verbindungen dieser Substanzklasse ist X eine Gruppe ausgewählt aus

| |
|---|
| 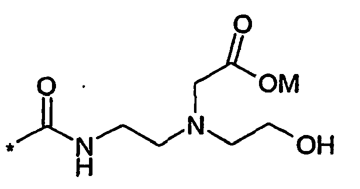 |
| 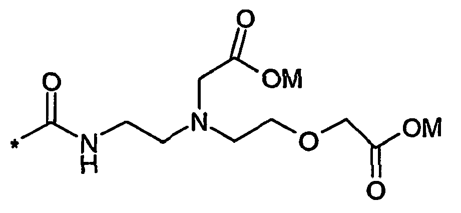 |
| 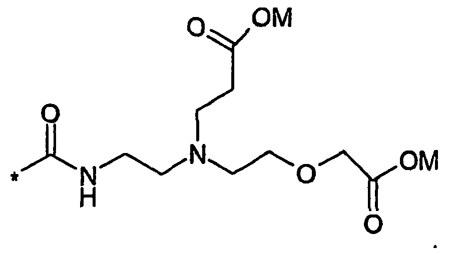 |
| -[(C(=O)-NH-(CH₂)₍₁₋₈₎]_{(0 oder 1)}-N⁺R¹R²-O⁻, wobei R¹ und R² jeweils unabhängig voneinander stehen für einen C1-8-Alkylrest, vorzugsweise |
| Methyl oder Ethyl |
| -NH-CH₂-COOM ; -NH-CH₂-CH₂-COOM |
| 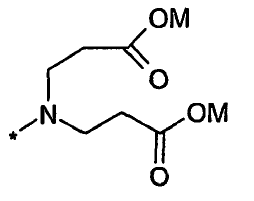 |
| -[(C(=O)-NH-(CH₂)₍₁₋₈₎]_{(0 oder 1)}-N⁺R¹R²-CH₂-COO⁻, wobei R¹ und R² jeweils unabhängig voneinander stehen für einen C1-8-Alkylrest, vorzugsweise Methyl oder Ethyl |
| -C(=O)-NH-(CH₂)₁₋₃-N⁺R¹R²-CH₂-CH(OH)-CH₂-(O)_{(0 oder 1)}-(S oder P)O₃⁻, wobei R¹ und R² jeweils unabhängig voneinander stehen für einen C1-8-Alkylrest, vorzugsweise Methyl oder Ethyl |

Zu den insbesondere bevorzugten Verbindungen gehören dabei die in der folgenden Tabelle gezeigten Verbindungen:

| |
|---|
| CF₃-O-(CH₂)₄-COOH, CF₃-O-(CH₂)₄-SO₃H; CF₃-O-(CH₂)₄-O-SO₃H |
| CF₃-O-(CH₂)₅-COOH; CF₃-O-(CH₂)₅-SO₃H; CF₃-O-(CH₂)₅-O-SO₃H |
| CF₃-O-(CH₂)₆-COOH; CF₃-O-(CH₂)₆-SO₃H, CF₃-O-(CH₂)₆-O-SO₃H |
| CF₃-O-(CH₂)₇-COOH; CF₃-O-(CH₂)₇-SO₃H; CF₃-O-(CH₂)₇-O-SO₃H |
| CF₃-O-(CH₂)₈-COOH; CF₃-O-(CH₂)₈-SO₃H; CF₃-O-(CH₂)₈-O-SO₃H |
| CF₃-O-(CH₂)₉-COOH; CF₃-O-(CH₂)₉-SO₃H; CF₃-O-(CH₂)₉-O-SO₃H |
| CF₃-O-(CH₂)₁₀-COOH; CF₃-O-(CH₂)₁₀-SO₃H; CF₃-O-(CH₂)₁₀-O-SO₃H |
| CF₃-O-(CH₂)₁₁-COOH; CF₃-O-(CH₂)₁₁-SO₃H; CF₃-O-(CH₂)₁₁-O-SO₃H |
| CF₃-O-(CH₂)₁₂-COOH; CF₃-O-(CH₂)₁₂-SO₃H; CF₃-O-(CH₂)₁₂-O-SO₃H |
| CF₃-O-(CH₂)₁₃-COOH; CF₃-O-(CH₂)₁₃-SO₃H; CF₃-O-(CH₂)₁₃-O-SO₃H |
| CF₃-O-(CH₂)₁₄-COOH; CF₃-O-(CH₂)₁₄-SO₃H; CF₃-O-(CH₂)₁₄-O-SO₃H |
| CF₃-O-(CH₂)₁₅-COOH; CF₃-O-(CH₂)₁₅-SO₃H; CF₃-O-(CH₂)₁₅-O-SO₃H |
| CF₃-O-(CH₂)₁₆-COOH; CF₃-O-(CH₂)₁₆-SO₃H; CF₃-O-(CH₂)₁₆-O-SO₃H |
| CF₃-O-(CH₂)₁₇-COOH; CF₃-O-(CH₂)₁₇-SO₃H; CF₃-O-(CH₂)₁₇-SO₃H |
| CF₃-O-(CH₂)₁₈-COOH, CF₃-O-(CH₂)₁₈-SO₃H; CF₃-O-(CH₂)₁₈-O-SO₃H |
| CF₃-O-(CH₂)₁₉-COOH; CF₃-O-(CH₂)₁₉-SO₃H; CF₃-O-(CH₂)₁₉-O-SO₃H |
| CF₃-O-(CH₂)₂₀-COOH; CF₃-O-(CH₂)₂₀-SO₃H; CF3-O-(CH₂)₂₀-O-SO₃H |
| CF₃-O-(CH₂)₂₁-COOH; CF₃-O-(CH₂)₂₁-SO₃H; CF₃-O-(CH₂)₂₁-O-SO₃H |
| CF₃-O-(CH₂)₂₂-COOH; CF₃-O-(CH₂)₂₂-SO₃H; CF₃-O-(CH₂)₂₂-O-SO₃H |
| CF₃-O-(CH₂)₂₃-COOH; CF₃-O-(CH₂)₂₃-SO₃H; CF₃-O-(CH₂)₂₃-O-SO₃H |
| CF₃-O-(CH₂)₂₄-COOH; CF₃-O-(CH₂)₂₄-SO₃H; CF₃-O-(CH₂)₂₄-O-SO₃H |
| SF₅-(CH₂)₄-COOH; SF₅-(CH₂)₄-SO₃H; SF₅-(CH₂)₄-O-SO₃H |
| SF₅-(CH₂)₅-COOH; SF₅-(CH₂)₅-SO₃H; SF₅-(CH₂)₅-O-SO₃H |
| SF₅-(CH₂)₆-COOH; SF₅-(CH₂)₆-SO₃H, SF₅-(CH₂)₆-O-SO₃H |
| SF₅-(CH₂)₇-COOH; SF₅-(CH₂)₇-SO₃H; SF₅-(CH₂)₇-O-SO₃H |
| SF₅-(CH₂)₈-COOH; SF₅-(CH₂)₈-SO₃H; SF₅-(CH₂)₈-O-SO₃H |
| SF₅-(CH₂)₉-COOH; SF₅-(CH₂)₉-SO₃H; SF₅-(CH₂)₉-O-SO₃H |
| SF₅-(CH₂)₁₀-COOH; SF₆-(CH₂)₁₀-SO₃H; SF₅-(CH₂)₁₀-O-SO₃H |
| SF₅-(CH₂)₁₁-COOH; SF₅-(CH₂)₁₁-SO₃H; SF₅-(CH₂)₁₁-O-SO₃H |
| SF₅-(CH₂)₁₂-COOH, SF₅-(CH₂)₁₂-SO₃H; SF₅-(CH₂)₁₂-O-SO₃H |
| SF₅-(CH₂)₁₃-COOH; SF₅-(CH₂)₁₃-SO₃H; SF₅-(CH₂)₁₃-O-SO₃H |
| SF₅-(CH₂)₁₄-COOH; SF₅-(CH₂)₁₄-SO₃H; SF₅-(CH₂)₁₄-O-SO₃H |
| SF₅-(CH₂)₁₅-COOH; SF₅-(CH₂)₁₅-SO₃H, SF₅-(CH₂)₁₅-O-SO₃H |
| SF₅-(CH₂)₁₆-COOH; SF₅-(CH₂)₁₆-SO₃H; SF₅-(CH₂)₁₆-O-SO₃H |
| SF₅-(CH₂)₁₇-COOH; SF₅-(CH₂)₁₇-SO₃H; SF₅-(CH₂)₁₇-O-SO₃H |
| SF₅-(CH₂)₁₈-COOH; SF₅-(CH₂)₁₈-SO₃H; SF₅-(CH₂)₁₈-O-SO₃H |
| SF₅-(CH₂)₁₉-COOH; SF₅-(CH₂)₁₉-SO₃H; SF₅-(CH₂)₁₉-O-SO₃H |
| SF₅-(CH₂)₂₀-COOH; SF₅-(CH₂)₂₀-SO₃H; SF₅-(CH₂)₂₀-O-SO₃H |
| SF₅-(CH₂)₂₁-COOH; SF₅-(CH₂)₂₁-SO₃H; SF₅-(CH₂)₂₁-O-SO₃H |
| SF₅-(CH₂)₂₂-COOH, SF₅-(CH₂)₂₂-SO₃H; SF₅-(CH₂)₂₂-O-SO₃H |
| SF₅-(CH₂)₂₃-COOH; SF₅-(CH₂)₂₃-SO₃H; SF₅-(CH₂)₂₃-O-SO₃H |
| SF₅-(CH₂)₂₄-COOH; SF₅-(CH₂)₂₄-SO₃H; SF₅-(CH₂)₂₄-O-SO₃H |
| SF₅-CH=CH-(CH₂)₄-COOH; SF₅-CH=CH-(CH₂)₄-SO₃H; SF₅-CH=CH-(CH₂)₄-O-SO₃H |
| SF₅-CH=CH-(CH₂)₅-COOH; SF₅-CH=CH-(CH₂)₅-SO₃H; SF₅-CH=CH-(CH₂)₅-O-SO₃H |
| SF₅-CH=CH-(CH₂)₈-COOH; SF₅-CH=CH-(CH₂)₆-SO₃H, SF₅-CH=CH-(CH₂)₆-O-SO₃H |
| SF₅-CH=CH-(CH₂)₇-COOH; SF₅-CH=CH-(CH₂)₇-SO₃H; SF₅-CH=CH-(CH₂)₇-O-SO₃H |
| SF₅-CH=CH-(CH₂)₈-COOH; SF₅-CH=CH-(CH₂)₈-SO₃H; SF₅-CH=CH-(CH₂)₈-O-SO₃H |
| SF₅-CH=CH-(CH₂)₉-COOH; SF₅-CH=CH-(CH₂)₉-SO₃H; SF₅-CH=CH-(CH₂)₉-O-SO₃H |
| SF₅-CH=CH-(CH₂)₁₀-COOH; SF₅-CH=CH-(CH₂)₁₀-SO₃H, SF₅-CH=CH-(CH₂)₁₀-O-SO₃H |
| SF₅-CH=CH-(CH₂)₁₁-COOH; SF₅-CH=CH-(CH₂)₁₁-SO₃H; SF₅-CH=CH-(CH₂)₁₁-O-SO₃H |
| SF₅-CH=CH-(CH₂)₁₂-COOH; SF₅-CH=CH-(CH₂)₁₂-SO₃H; SF₅-CH=CH-(CH₂)₁₂-O-SO₃H |
| SF₅-CH=CH-(CH₂)₁₃-COOH; SF₅-CH=CH-(CH₂)₁₃-SO₃H; SF₅-CH=CH-(CH₂)₁₃-O-SO₃H |
| SF₅-CH=CH-(CH₂)₁₄-COOH; SF₅-CH=CH-(CH₂)₁₄-SO₃H; SF₅-CH=CH-(CH₂)₁₄-O-SO₃H |
| SF₅-CH₌CH-(CH₂)₁₅-COOH; SF₅-CH=CH-(CH₂)₁₅-SO₃H; SF₅-CH=CH-(CH₂)₁₅-O-SO₃H |
| SF₅-CH=CH-(CH₂)₁₆-COOH; SF₅-CH=CH-(CH₂)₁₆-SO₃H, SF₅-CH=CH-(CH₂)₁₆-O-SO₃H |
| SF₅-CH=CH-(CH₂)₁₇-COOH; SF₅-CH=CH-(CH₂)₁₇-SO₃H; SF₅-CH=CH-(CH₂)₁₇-O-SO₃H |
| SF₅-CH=CH-(CH₂)₁₈-COOH; SF₅-CH=CH-(CH₂)₁₈-SO₃H; SF₅-CH=CH-(CH₂)₁₈-O-SO₃H |
| SF₅-CH=CH-(CH₂)₁₉-COOH; SF₅-CH=CH-(CH₂)₁₉-SO₃H, SF₅-CH=CH-(CH₂)₁₉-O-SO₃H |
| SF₅-CH=CH-(CH₂)₂₀-COOH, SF₅-CH=CH-(CH₂)₂₀-SO₃H; SF₅-CH=CH-(CH₂)₂₀-O-SO₃H |
| SF₅-CH=CH-(CH₂)₂₁-COOH, SF₅-CH=CH-(CH₂)₂₁-SO₃H; SF₅-CH=CH-(CH₂)₂₁-O-SO₃H |
| SF₅-CH=CH-(CH₂)₂₂-COOH; SF₅-CH=CH-(CH₂)₂₂-SO₃H; SF₅-CH=CH-(CH₂)₂₂-O-SO₃H |
| SF₅-CH=CH-(CH₂)₂₃-COOH; SF₅-CH=CH-(CH₂)₂₃-SO₃H; SF₅-CH=CH-(CH₂)₂₃-O-SO₃H |
| SF₅-CH=CH-(CH₂)₂₄-COOH; SF₅-CH=CH-(CH₂)₂₄-SO₃H; SF₅-CH=CH-(CH₂)₂₄-O-SO₃H |
| SF₅-CH₂CHBr-(CH₂)₄-COOH; SF₅-CH₂CHBr-(CH₂)₄-SO₃H; SF₅-CH₂CHBr-(CH₂)₄-O-SO₃H |
| SF₅-CH₂CHBr-(CH₂)₅-COOH, SF₅-CH₂CHBr-(CH₂)₅-SO₃H; SF₅-CH₂CHBr-(CH₂)₅-O-SO₃H |
| SF₅-CH₂CHBr-(CH₂)₆-COOH; SF₅-CH₂CHBr-(CH₂)₆-SO₃H; SF₅-CH₂CHBr-(CH₂)₆-O-SO₃H |
| SF₅-CH₂CHBr-(CH₂)₇-COOH-, SF₅-CH₂CHBr-(CH₂)₇-SO₃H; SF₅-CH₂CHBr-(CH₂)₇-O-SO₃H |
| SF₅-CH₂CHBr-(CH₂)₈-COOH; SF₅-CH₂CHBr-(CH₂)₈-SO₃H; SF₅-CH₂CHBr-(CH₂)₈-O-SO₃H |
| SF₅-CH₂CHBr-(CH₂)₉-COOH; SF₅-CH₂CHBr-(CH₂)₉-SO₃H; SF₅-CH₂CHBr-(CH₂)₉-O-SO₃H |
| SF₅-CH₂CHBr-(CH₂)₁₀-COOH; SF₅-CH₂CHBr-(CH₂)₁₀-SO₃H; SF₅-CH₂CHBr-(CH₂)₁₀-O-SO₃H |
| SF₅-CH₂CHBr-(CH₂)₁₁-COOH; SF₅-CH₂CHBr-(CH₂)₁₁-SO₃H; SF₅-CH₂CHBr-(CH₂)₁₁-O-SO₃H |
| SF₅-CH₂CHBr-(CH₂)₁₂-COOH; SF₅-CH₂CHBr-(CH₂)₁₂-SO₃H; SF₁₅-CH₂CHBr-(CH₂)₁₂-O-SO₃H |
| SF₅-CH₂CHBr-(CH₂)₁₃-COOH; SF₅-CH₂CHBr-(CH₂)₁₃-SO₃H; SF₅-CH₂CHBr-(CH₂)₁₃-O-SO₃H |
| SF₅-CH₂CHBr-(CH₂)₁₄-COOH; SF₅-CH₂CHBr-(CH₂)₁₄-SO₃H; SF₅-CH₂CHBr-(CH₂)₁₄-O-SO₃H |
| SF₅-CH₂CHBr-(CH₂)₁₅-COOH; SF₅-CH₂CHBr-(CH₂)₁₅-SO₃H; SF₅-CH₂CHBr-(CH₂)₁₅-O-SO₃H |
| SF₅-CH₂CHBr-(CH₂)₁₀-COOH; SF₅-CH₂CHBr-(CH₂)₁₆-SO₃H; SF₅-CH₂CHBr-(CH₂)₁₆-O-SO₃H |
| SF₅-CH₂CHBr-(CH₂)₁₇-COOH; SF₅-CH₂CHBr-(CH₂)₁₇-SO₃H; SF₅-CH₂CHBr-(CH₂)₁₇-O-SO₃H |
| SF₅-CH₂CHBr-(CH₂)₁₈-COOH; SF₅-CH₂CHBr-(CH₂)₁₈-SO₃H; SF₅-CH₂CHBr-(CH₂)₁₈-O-SO₃H |
| SF₅-CH₂CHBr-(CH₂)₁₉-COOH; SF₅-CH₂CHBr-(CH₂)₁₉-SO₃H; SF₅-CH₂CHBr-(CH₂)₁₉-O-SO₃H |
| SF₅-CH₂CHBr-(CH₂)₂₀-COOH; SF₅-CH₂CHBr-(CH₂)₂₀-SO₃H; SF₅-CH₂CHBr-(CH₂)₂₀-O-SO₃H |
| SF₅-CH₂CHBr-(CH₂)₂₁-COOH; SF₅-CH₂CHBr-(CH₂)₂₁-SO₃H; SF₅-CH₂CHBr-(CH₂)₂₁-O-SO₃H |
| SF₅-CH₂CHBr-(CH₂)₂₂-COOH; SF₅-CH₂CHBr-(CH₂)₂₂-SO₃H; SF₅-CH₂CHBr-(CH₂)₂₂-O-SO₃H |
| SF₅-CH₂CHBr-(CH₂)₂₃-COOH; SF₅-CH₂CHBr-(CH₂)₂₃-SO₃H; SF₅-CH₂CHBr-(CH₂)₂₃-O-SO₃H |
| SF₅-CH₂CHBr-(CH₂)₂₄-COOH; SF₅-CH₂CHBr-(CH₂)₂₄-SO₃H, SF₅-CH₂CHBr-(CH₂)₂₄-O-SO₃H |
| CF₃-O-(CH₂)₄-OH; CF₃-O-(CH₂)₄-(OCH₂CH₂)ₘ-OH; CF₃-O-(CH₂)₄-(OCH₂CH₂)ₘ-SO₂-CH=CH₂ |
| CF₃-O-(CH₂)₅-OH; CF₃-O-(CH₂)₅-(OCH₂CH₂)ₘ-OH; CF₃-O-(CH₂)₅-(OCH₂CH₂)ₘ-SO₂-CH=CH₂ |
| CF₃-O-(CH₂)₆-OH; CF₃-O-(CH₂)₆-(OCH₂CH₂)ₘ-OH; CF₃-O-(CH₂)₆-(OCH₂CH₂)ₘ-SO₂-CH=CH₂ |
| CF₃-O-(CH₂)₇-OH; CF₃-O-(CH₂)₇-(OCH₂CH₂)ₘ-OH; CF₃-O-(CH₂)₇-(OCH₂CH₂)ₘ-SO₂-CH=CH₂ |
| CF₃-O-(CH₂)₈-OH; CF₃-O-(CH₂)₈-(OCH₂CH₂)ₘ-OH; CF₃-O-(CH₂)₈-(OCH₂CH₂)ₘ-SO₂-CH=CH₂ |
| CF₃-O-(CH₂)₉-OH; CF₃-O-(CH₂)₉-(OCH₂CH₂)ₘ-OH; CF₃-O-(CH₂)₉-(OCH₂CH₂)ₘ-SO₂-CH=CH₂ |
| CF₃-O-(CH₂)₁₀-OH; CF₃-O-(CH₂)₁₀-(OCH₂CH₂)ₘ-OH; CF₃-O-(CH₂)₁₀-(OCH₂CH₂)ₘ-SO₂-CH=CH₂ |
| CF₃-O-(CH₂)₁₁-OH; CF₃-O-(CH₂)₁₁-(OCH₂CH₂)ₘ-OH; CF₃-O-(CH₂)₁₁-(OCH₂CH₂)ₘ-SO₂-CH=CH₂ |
| CF₃-O-(CH₂)₁₂-OH; CF₃-O-(CH₂)₁₂-(OCH₂CH₂)ₘ-OH; CF₃-O-(CH₂)₁₂-(OCH₂CH₂)ₘ-SO₂-CH=CH₂ |
| CF₃-O-(CH₂)₁₃-OH; CF₃-O-(CH₂)₁₃-(OCH₂CH₂)ₘ-OH; CF₃-O-(CH₂)₁₃-(OCH₂CH₂)ₘ-SO₂-CH=CH₂ |
| CF₃-O-(CH₂)₁₄-OH; CF₃-O-(CH₂)₁₄-(OCH₂CH₂)ₘ-OH; CF₃-O-(CH₂)₁₄-(OCH₂CH₂)ₘ-SO₂-CH=CH₂ |
| CF₃-O-(CH₂)₁₅-OH; CF₃-O-(CH₂)₁₅-(OCH₂CH₂)ₘ-OH; CF₃-O-(CH₂)₁₅-(OCH₂CH₂)ₘ-SO₂-CH=CH₂ |
| CF₃-O-(CH₂)₁₆-OH; CF₃-O-(CH₂)₁₆-(OCH₂CH₂)ₘ-OH; CF₃-O-(CH₂)₁₆-(OCH₂CH₂)ₘ-SO₂-CH=CH₂ |
| CF₃-O-(CH₂)₁₇-OH; CF₃-O-(CH₂)₁₇-(OCH₂CH₂)ₘ-OH; CF₃-O-(CH₂)₁₇-(OCH₂CH₂)ₘ-SO₂-CH=CH₂ |
| CF₃-O-(CH₂)₁₈-OH; CF₃-O-(CH₂)₁₈-(OCH₂CH₂)ₘ-OH; CF₃-O-(CH₂)₁₈-(OCH₂CH₂)ₘ-SO₂-CH=CH₂ |
| CF₃-O-(CH₂)₁₉-OH; CF₃-O-(CH₂)₁₉-(OCH₂CH₂)ₘ-OH; CF₃-O-(CH₂)₁₉-(OCH₂CH₂)ₘ-SO₂-CH=CH₂ |
| CF₃-O-(CH₂)₂₀-OH; CF₃-O-(CH₂)₂₀-(OCH₂CH₂)ₘ-OH; CF₃-O-(CH₂)₂₀-(OCH₂CH₂)ₘ-SO₂-CH=CH₂ |
| CF₃-O-(CH₂)₂₁-OH; CF₃-O-(CH₂)₂₁-(OCH₂CH₂)ₘ-OH; CF₃-O-(CH₂)₂₁-(OCH₂CH₂)ₘ-SO₂-CH=CH₂ |
| CF₃-O-(CH₂)₂₂-OH; CF₃-O-(CH₂)₂₂-(OCH₂CH₂)ₘ-OH; CF₃-O-(CH₂)₂₂-(OCH₂CH₂)ₘ-SO₂-CH=CH₂ |
| CF₃-O-(CH₂)₂₃-OH; CF₃-O-(CH₂)₂₃-(OCH₂CH₂)ₘ-OH; CF₃-O-(CH₂)₂₃-(OCH₂CH₂)ₘ-SO₂-CH=CH₂ |
| CF₃-O-(CH₂)₂₄-OH; CF₃-O-(CH₂)₂₄-(OCH₂CH₂)ₘ-OH; CF₃-O-(CH₂)₂₄-(OCH₂CH₂)ₘ-SO₂-CH=CH₂ |
| SF₅-(CH₂)₄-OH; SF₅-(CH₂)₄-(OCH₂CH₂)ₘ-OH; SF₅-(CH₂)₄-(OCH₂CH₂)ₘ-SO₂-CH=CH₂ |
| SF₅-(CH₂)₅-OH, SF₅-(CH₂)₅-(OCH₂CH₂)ₘ-OH; SF₅-(CH₂)₅-(OCH₂CH₂)ₘ-SO₂-CH=CH₂ |
| SF₅-(CH₂)₆-OH; SF₅-(CH₂)₆-(OCH₂CH₂)ₘ-OH; SF₅-(CH₂)₆-(OCH₂CH₂)ₘ-SO₂-CH=CH₂ |
| SF₅-(CH₂)₇-OH; SF₅-(CH₂)₇-(OCH₂CH₂)ₘ-OH; SF₅-(CH₂)₇-(OCH₂CH₂)ₘ-SO₂-CH=CH₂ |
| SF₅-(CH₂)₈-OH, SF₅-(CH₂)₈-(OCH₂CH₂)ₘ-OH; SF₅-(CH₂)₈-(OCH₂CH₂)ₘ-SO₂-CH=CH₂ |
| SF₅-(CH₂)₉-OH; SF₅-(CH₂)₉-(OCH₂CH₂)ₘ-OH; SF₅-(CH₂)₉-(OCH₂CH₂)ₘ-SO₂-CH=CH₂ |
| SF₅-(CH₂)₁₀-OH; SF₅-(CH₂)₁₀-(OCH₂CH₂)ₘ-OH; SF₅-(CH₂)₁₀-(OCH₂CH₂)ₘ-SO₂-CH=CH₂ |
| SF₅-(CH₂)₁₁-OH; SF₅-(CH₂)₁₁-(OCH₂CH₂)ₘ-OH; SF₅-(CH₂)₁₁-(OCH₂CH₂)ₘ-SO₂-CH=CH₂ |
| SF₅-(CH₂)₁₂-OH; SF₅-(CH₂)₁₂-(OCH₂CH₂)ₘ-OH; SF₅-(CH₂)₁₂-(OCH₂CH₂)ₘ-SO₂-CH=CH₂ |
| SF₅-(CH₂)₁₃-OH; SF₅-(CH₂)₁₃-(OCH₂CH₂)ₘ-OH; SF₅-(CH₂)₁₃-(OCH₂CH₂)ₘ-SO₂-CH=CH₂ |
| SF₅-(CH₂)₁₄-OH; SF₅-(CH₂)₁₄-(OCH₂CH₂)ₘ-OH; SF₅-(CH₂)₁₄-(OCH₂CH₂)ₘ-SO₂-CH=CH₂ |
| SF₅-(CH₂)₁₅-OH; SF₅-(CH₂)₁₅-(OCH₂CH₂)ₘ-OH; SF₅-(CH₂)₁₅-(OCH₂CH₂)ₘ-SO₂-CH=CH₂ |
| SF₅-(CH₂)₁₆-OH; SF₅-(CH₂)₁₆-(OCH₂CH₂)ₘ-OH; SF₅-(CH₂)₁₆-(OCH₂CH₂)ₘ-SO₂-CH=CH₂ |
| SF₅-(CH₂)₁₇-OH; SF₅-(CH₂)₁₇-(OCH₂CH₂)ₘ-OH; SF₅-(CH₂)₁₇-(OCH₂CH₂)ₘ-SO₂-CH=CH₂ |
| SF₅-(CH₂)₁₈-OH; SF₅-(CH₂)₁₈-(OCH₂CH₂)ₘ-OH; SF₅-(CH₂)₁₈-(OCH₂CH₂)ₘ-SO₂-CH=CH₂ |
| SF₅-(CH₂)₁₉-OH; SF₅-(CH₂)₁₉-(OCH₂CH₂)ₘ-OH; SF₅-(CH₂)₁₉-(OCH₂CH₂)ₘ-SO₂-CH=CH₂ |
| SF₅-(CH₂)₂₀-OH; SF₅-(CH₂)₂₀-(OCH₂CH₂)ₘ-OH; SF₅-(CH₂)₂₀-(OCH₂CH₂)ₘ-SO₂-CH=CH₂ |
| SF₅-(CH₂)₂₁-OH; SF₅-(CH₂)₂₁-(OCH₂CH₂)ₘ-OH; SF₅-(CH₂)₂₁-(OCH₂CH₂)ₘ-SO₂-CH=CH₂ |
| SF₅-(CH₂)₂₂-OH; SF₅-(CH₂)₂₂-(OCH₂CH₂)ₘ-OH; SF₅-(CH₂)₂₂-(OCH₂CH₂)ₘ-SO₂-CH=CH₂ |
| SF₅-(CH₂)₂₃-OH; SF₅-(CH₂)₂₃-(OCH₂CH₂)ₘ-OH; SF₅-(CH₂)₂₃-(OCH₂CH₂)ₘ-SO₂-CH=CH₂ |
| SF₅-(CH₂)₂₄-OH; SF₅-(CH₂)₂₄-(OCH₂CH₂)ₘ-OH; SF₅-(CH₂)₂₄-(OCH₂CH₂)ₘ-SO₂-CH=CH₂ |
| SF₅-CH=CH-(CH₂)₄-OH; SF₅-CH=CH-(CH₂)₄-(OCH₂CH₂)ₘ-OH; SF₅-CH=CH-(CH₂)₄-(OCH₂CH₂)ₘ-SO₂-CH=CH₂ |
| SF₅-CH=CH-(CH₂)₅-OH; SF₅-CH=CH-(CH₂)₅-(OCH₂CH₂)ₘ-OH; SF₅-CH=CH-(CH₂)₅-(OCH₂CH₂)ₘ-SO₂-CH=CH₂ |
| SF₅-CH=CH-(CH₂)₆-OH; SF₅-CH=CH-(CH₂)₆-(OCH₂CH₂)ₘ-OH; SF₅-CH=CH-(CH₂)₆-(OCH₂CH₂)ₘ-SO₂-CH=CH₂ |
| SF₅-CH=CH-(CH₂)₇-OH; SF₅-CH=CH-(CH₂)₇-(OCH₂CH₂)ₘ-OH; SF₅-CH=CH-(CH₂)₇-(OCH₂CH₂)ₘ-SO₂-CH=CH₂ |
| SF₅-CH=CH-(CH₂)₈-OH; SF₅-CH=CH-(CH₂)₈-(OCH₂CH₂)ₘ-OH; SF₅-CH=CH-(CH₂)₈-(OCH₂CH₂)ₘ-SO₂-CH=CH₂ |
| SF₅-CH=CH-(CH₂)₉-OH; SF₅-CH=CH-(CH₂)₉-(OCH₂CH₂)ₘ-OH; SF₅-CH=CH-(CH₂)₉-(OCH₂CH₂)ₘ-SO₂-CH=CH₂ |
| SF₅-CH=CH-(CH₂)₁₀-OH; SF₅-CH=CH-(CH₂)₁₀-(OCH₂CH₂)ₘ-OH; SF₅-CH=CH-(CH₂)₁₀-(OCH₂CH₂)ₘ-SO₂-CH=CH₂ |
| SF₅-CH=CH-(CH₂)₁₁-OH; SF₅-CH=CH-(CH₂)₁₁-(OCH₂CH₂)ₘ-OH; SF₅-CH=CH-(CH₂)₁₁-(OCH₂CH₂)ₘ-SO₂-CH=CH₂ |
| SF₅-CH=CH-(CH₂)₁₂-OH; SF₅-CH=CH-(CH₂)₁₂-(OCH₂CH₂)ₘ-OH; SF₅-CH=CH-(CH₂)₁₂-(OCH₂CH₂)ₘ-SO₂-CH=CH₂ |
| SF₅-CH=CH-(CH₂)₁₃-OH; SF₅-CH=CH-(CH₂)₁₃-(OCH₂CH₂)ₘ-OH; SF₅-CH=CH-(CH₂)₁₃-(OCH₂CH₂)ₘ-SO₂-CH=CH₂ |
| SF₅-CH=CH-(CH₂)₁₄-OH; SF₅-CH=CH-(CH₂)₁₄-(OCH₂CH₂)ₘ-OH; SF₅-CH=CH-(CH₂)₁₄-(OCH₂CH₂)ₘ-SO₂-CH=CH₂ |
| SF₅-CH=CH-(CH₂)₁₅-OH; SF₅-CH=CH-(CH₂)₁₅-(OCH₂CH₂)ₘ-OH; SF₅-CH=CH-(CH₂)₁₅-(OCH₂CH₂)ₘ-SO₂-CH=CH₂ |
| SF₅-CH=CH-(CH₂)₁₆-OH; SF₅-CH=CH-(CH₂)₁₆-(OCH₂CH₂)ₘ-OH; SF₅-CH=CH-(CH₂)₁₆-(OCH₂CH₂)ₘ-SO₂-CH=CH₂ |
| SF₅-CH=CH-(CH₂)₁₇-OH; SF₅-CH=CH-(CH₂)₁₇-(OCH₂CH₂)ₘ-OH; SF₅-CH=CH-(CH₂)₁₇-(OCH₂CH₂)ₘ-SO₂-CH=CH₂ |
| SF₅-CH=CH-(CH₂)₁₈-OH; SF₅-CH=CH-(CH₂)₁₈-(OCH₂CH₂)ₘ-OH; SF₅-CH=CH-(CH₂)₁₈-(OCH₂CH₂)ₘ-SO₂-CH=CH₂ |
| SF₅-CH=CH-(CH₂)₁₉-OH; SF₅-CH=CH-(CH₂)₁₉-(OCH₂CH₂)ₘ-OH; SF₅-CH=CH-(CH₂)₁₉-(OCH₂CH₂)ₘ-SO₂-CH=CH₂ |
| SF₅-CH=CH-(CH₂)₂₀-OH; SF₅-CH=CH-(CH₂)₂₀-(OCH₂CH₂)ₘ-OH; SF₅-CH=CH-(CH₂)₂₀-(OCH₂CH₂)ₘ-SO₂-CH=CH₂ |
| SF₅-CH=CH-(CH₂)₂₁-OH; SF₅-CH=CH-(CH₂)₂₁-(OCH₂CH₂)ₘ-OH; SF₅-CH=CH-(CH₂)₂₁-(OCH₂CH₂)ₘ-SO₂-CH=CH₂ |
| SF₅-CH=CH-(CH₂)₂₂-OH; SF₅-CH=CH-(CH₂)₂₂-(OCH₂CH₂)ₘ-OH; SF₅-CH=CH-(CH₂)₂₂-(OCH₂CH₂)ₘ-SO₂-CH=CH₂ |
| SF₅-CH=CH-(CH₂)₂₃-OH; SF₅-CH=CH-(CH₂)₂₃-(OCH₂CH₂)ₘ-OH; SF₅-CH=CH-(CH₂)₂₃-(OCH₂CH₂)ₘ-SO₂-CH=CH₂ |
| SF₅-CH=CH-(CH₂)₂₄-OH; SF₅-CH=CH-(CH₂)₂₄-(OCH₂CH₂)ₘ-OH; SF₅-CH=CH-(CH₂)₂₄-(OCH₂CH₂)ₘ-SO₂-CH=CH₂ |
| SF₅-CH₂CHBr-(CH₂)₄-OH; SF₅-CH₂CHBr-(CH₂)₄-(OCH₂CH₂)ₘ-OH; SF₅-CH₂CHBr-(CH₂)₄-(OCH₂CH₂)ₘ-SO₂-CH=CH₂ |
| SF₅-CH₂CHBr-(CH₂)₅-OH, SF₅-CH₂CHBr-(CH₂)₅-(OCH₂CH₂)ₘ-OH, SF₅-CH₂CHBr-(CH₂)₅-(OCH₂CH₂)ₘ-SO₂-CH=CH₂ |
| SF₅-CH₂CHBr-(CH₂)₆-OH; SF₅-CH₂CHBr-(CH₂)₆-(OCH₂CH₂)ₘ-OH; SF₅-CH₂CHBr-(CH₂)₆-(OCH₂CH₂)ₘ-SO₂-CH=CH₂ |
| SF₅-CH₂CHBr-(CH₂)₇-OH; SF₅-CH₂CHBr-(CH₂)₇-(OCH₂CH₂)ₘ-OH; SF₅-CH₂CHBr-(CH₂)₇-(OCH₂CH₂)ₘ-SO₂-CH=CH₂ |
| SF₅-CH₂CHBr-(CH₂)₈-OH; SF₅-CH₂CHBr-(CH₂)₈-(OCH₂CH₂)ₘ-OH; SF₅-CH₂CHBr-(CH₂)₈-(OCH₂CH₂)ₘ-SO₂-CH=CH₂ |
| SF₅-CH₂CHBr-(CH₂)₉-OH; SF₅-CH₂CHBr-(CH₂)₉-(OCH₂CH₂)ₘ-OH; SF₅-CH₂CHBr-(CH₂)₉-(OCH₂CH₂)ₘ-SO₂-CH=CH₂ |
| SF₅-CH₂CHBr-(CH₂)₁₀-OH; SF₅-CH₂CHBr-(CH₂)₁₀-(OCH₂CH₂)ₘ-OH; SF₅-CH₂CHBr-(CH₂)₁₀-(OCH₂CH₂)ₘ-SO₂-CH=CH₂ |
| SF₅-CH₂CHBr-(CH₂)₁₁-OH; SF₅-CH₂CHBr-(CH₂)₁₁-(OCH₂CH₂)ₘ-OH; SF₅-CH₂CHBr-(CH₂)₁₁-(OCH₂CH₂)ₘ-SO₂-CH=CH₂ |
| SF₅-CH₂CHBr-(CH₂)₁₂-OH; SF₅-CH₂CHBr-(CH₂)₁₂-(OCH₂CH₂)ₘ-OH; SF₅-CH₂CHBr-(CH₂)₁₂-(OCH₂CH₂)ₘ-SO₂-CH=CH₂ |
| SF₅-CH₂CHBr-(CH₂)₁₃-OH; SF₅-CH₂CHBr-(CH₂)₁₃-(OCH₂CH₂)ₘ-OH; SF₅-CH₂CHBr-(CH₂)₁₃-(OCH₂CH₂)ₘ-SO₂-CH=CH₂ |
| SF₅-CH₂CHBr-(CH₂)₁₄-OH; SF₅-CH₂CHBr-(CH₂)₁₄-(OCH₂CH₂)ₘ-OH; SF₅-CH₂CHBr-(CH₂)₁₄-(OCH₂CH₂)ₘ-SO₂-CH=CH₂ |
| SF₅-CH₂CHBr-(CH₂)₁₅-OH; SF₅-CH₂CHBr-(CH₂)₁₅-(OCH₂CH₂)ₘ-OH; SF₅-CH₂CHBr-(CH₂)₁₅-(OCH₂CH₂)ₘ-SO₂-CH=CH₂ |
| SF₅-CH₂CHBr-(CH₂)₁₆-OH, SF₅-CH₂CHBr-(CH₂)₁₆-(OCH₂CH₂)ₘ-OH; SF₅-CH₂CHBr-(CH₂)₁₆-(OCH₂CH₂)ₘ-SO₂-CH=CH₂ |
| SF₅-CH₂CHBr-(CH₂)₁₇-OH; SF₅-CH₂CHBr-(CH₂)₁₇-(OCH₂CH₂)ₘ-OH; SF₅-CH₂CHBr-(CH₂)₁₇-(OCH₂CH₂)ₘ-SO₂-CH=CH₂ |
| SF₅-CH₂CHBr-(CH₂)₁₈-OH; SF₅-CH₂CHBr-(CH₂)₁₈-(OCH₂CH₂)ₘ-OH; SF₅-CH₂CHBr-(CH₂)₁₈-(OCH₂CH₂)ₘ-SO₂-CH=CH₂ |
| SF₅-CH₂CHBr-(CH₂)₁₉-OH; SF₅-CH₂CHBr-(CH₂)₁₉-(OCH₂CH₂)m-OH; SF₅-CH₂CHBr-(CH₂)₁₉-(OCH₂CH₂)ₘ-SO₂-CH=CH₂ |
| SF₅-CH₂CHBr-(CH₂)₂₀-OH; SF₅-CH₂CHBr-(CH₂)₂₀-(OCH₂CH₂)ₘ-OH; SF₅-CH₂CHBr-(CH₂)₂₀-(OCH₂CH₂)ₘ-SO₂-CH=CH₂ |
| SF₅-CH₂CHBr-(CH₂)₂₁-OH; SF₅-CH₂CHBr-(CH₂)₂₁-(OCH₂CH₂)ₘ-OH; SF₅-CH₂CHBr-(CH₂)₂₁-(OCH₂CH₂)ₘ-SO₂-CH=CH₂ |
| SF₅-CH₂CHBr-(CH₂)₂₂-OH; SF₅-CH₂CHBr-(CH₂)₂₂-(OCH₂CH₂)ₘ-OH; SF₅-CH₂CHBr-(CH₂)₂₂-(OCH₂CH₂)ₘ-SO₂-CH=CH₂ |
| SF₅-CH₂CHBr-(CH₂)₂₃-OH; SF₅-CH₂CHBr-(CH₂)₂₃-(OCH₂CH₂)ₘ-OH; SF₅-CH₂CHBr-(CH₂)₂₃-(OCH₂CH₂)ₘ-SO₂-CH=CH₂ |
| SF₅-CH₂CHBr-(CH₂)₂₄-OH; SF₅-CH₂CHBr-(CH₂)₂₄-(OCH₂CH₂)ₘ-OH; SF₅-CH₂CHBr-(CH₂)₂₄-(OCH₂CH₂)ₘ-SO₂-CH=CH₂ |
| CF₃-O-(CH₂)₄-(OCH₂CH₂)ₘ-OCOCH=CH₂; CF₃-O-(CH₂)₄-(OCH₂CH₂)ₘ-OCH=CH₂; CF₃-O-(CH₂)₄-(OCH₂CH₂)ₘ-OAr(NCO)ₚ |
| CF₃-O-(CH₂)₅-(OCH₂CH₂)ₘ-OCOCH=CH₂; CF₃-O-(CH₂)₅-(OCH₂CH₂)ₘ-OCH=CH₂; CF₃-O-(CH₂)₅-(OCH₂CH₂)ₘ-OAr(NCO)ₚ |
| CF₃-O-(CH₂)₆-(OCH₂CH₂)ₘ-OCOCH=CH₂; CF₃-O-(CH₂)₆-(OCH₂CH₂)ₘ-OCH=CH₂; CF₃-O-(CH₂)₆-(OCH₂CH₂)ₘ-OAr(NCO)ₚ |
| CF₃-O-(CH₂)₇-(OCH₂CH₂)ₘ-OCOCH=CH₂; CF₃-O-(CH₂)₇-(OCH₂CH₂)ₘ-OCH=CH₂; CF₃-O-(CH₂)₇-(OCH₂CH₂)ₘ-OAr(NCO)ₚ |
| CF₃-O-(CH₂)₈-(OCH₂CH₂)ₘ-OCOCH=CH₂; CF₃-O-(CH₂)₈-(OCH₂CH₂)ₘ-OCH=CH₂; CF₃-O-(CH₂)₈-(OCH₂CH₂)ₘ-OAr(NCO)ₚ |
| CF₃-O-(CH₂)₉-(OCH₂CH₂)ₘ-OCOCH=CH₂; CF₃-O-(CH₂)₉-(OCH₂CH₂)ₘ-OCH=CH₂; CF₃-O-(CH₂)₉-(OCH₂CH₂)ₘ-OAr(NCO)ₚ |
| CF₃-O-(CH₂)₁₀-(OCH₂CH₂)ₘ-OCOCH=CH₂; CF₃-O-(CH₂)₁₀-(OCH₂CH₂)ₘ-OCH=CH₂; CF₃-O-(CH₂)₁₀-(OCH₂CH₂)ₘ-OAr(NCO)ₚ |
| CF₃-O-(CH₂)₁₁-(OCH₂CH₂)ₘ-OCOCH=CH₂; CF₃-O-(CH₂)₁₁-(OCH₂CH₂)ₘ-OCH=CH₂; CF₃-O-(CH₂)₁₁-(OCH₂CH₂)ₘ-OAr(NCO)ₚ |
| CF₃-O-(CH₂)₁₂-(OCH₂CH₂)ₘ-OCOCH=CH₂; CF₃-O-(CH₂)₁₂(OCH₂CH₂)ₘ-OCH=CH₂; CF₃-O-(CH₂)₁₂-(OCH₂CH₂)ₘ-OAr(NCO)ₚ |
| CF₃-O-(CH₂)₁₃-(OCH₂CH₂)ₘ-OCOCH=CH₂; CF₃-O-(CH₂)₁₃-(OCH₂CH₂)ₘ-OCH=CH₂; CF₃-O-(CH₂)₁₃-(OCH₂CH₂)ₘ-OAr(NCO)ₚ |
| CF₃-O-(CH₂)₁₄-(OCH₂CH₂)ₘ-OCOCH=CH₂; CF₃-O-(CH₂)₁₄-(OCH₂CH₂)ₘ-OCH=CH₂; CF₃-O-(CH₂)₁₄-(OCH₂CH₂)ₘ-OAr(NCO)ₚ |
| CF₃-O-(CH₂)₁₅-(OCH₂CH₂)ₘ-OCOCH=CH₂; CF₃-O-(CH₂)₁₅-(OCH₂CH₂)ₘ-OCH=CH₂; CF₃-O-(CH₂)₁₅-(OCH₂CH₂)ₘ-OAr(NCO)ₚ |
| CF₃-O-(CH₂)₁₆-(OCH₂CH₂)ₘ-OCOCH=CH₂; CF₃-O-(CH₂)₁₆-(OCH₂CH₂)ₘ-OCH=CH₂; CF₃-O-(CH₂)₁₆-(OCH₂CH₂)ₘ-OAr(NCO)ₚ |
| CF₃-O-(CH₂)₁₇-(OCH₂CH₂)ₘ-OCOCH=CH₂; CF₃-O-(CH₂)₁₇-(OCH₂CH₂)ₘ-OCH=CH₂; CF₃-O-(CH₂)₁₇-(OCH₂CH₂)ₘ-OAr(NCO)ₚ |
| CF₃-O-(CH₂)₁₈-(OCH₂CH₂)ₘ-OCOCH=CH₂; CF₃-O-(CH₂)₁₈-(OCH₂CH₂)ₘ-OCH=CH₂; CF₃-O-(CH₂)₁₈-(OCH₂CH₂)ₘ-OAr(NCO)ₚ |
| CF₃-O-(CH₂)₁₉-(OCH₂CH₂)ₘ-OCOCH=CH₂; CF₃-O-(CH₂)₁₉-(OCH₂CH₂)ₘ-OCH=CH₂; CF₃-O-(CH₂)₁₉-(OCH₂CH₂)ₘ-OAr(NCO)ₚ |
| CF₃-O-(CH₂)₂₀-(OCH₂CH₂)ₘ-OCOCH=CH₂; CF₃-O-(CH₂)₂₀-(OCH₂CH₂)ₘ-OCH=CH₂; CF₃-O-(CH₂)₂₀-(OCH₂CH₂)ₘ-OAr(NCO)ₚ |
| CF₃-O-(CH₂)₂₁-(OCH₂CH₂)ₘ-OCOCH=CH₂; CF₃=O-(CH₂)₂₁-(OCH₂CH₂)ₘ-OCH=CH₂; CF₃-O-(CH₂)₂₁-(OCH₂CH₂)ₘ-OAr(NCO)ₚ |
| CF₃-O-(CH₂)₂₂-(OCH₂CH₂)ₘ-OCOCH=CH₂; CF₃-O-(CH₂)₂₂-(OCH₂CH₂)ₘ-OCH=CH₂; CF₃-O-(CH₂)₂₂-(OCH₂CH₂)ₘ-OAr(NCO)ₚ |
| CF₃-O-(CH₂)₂₃-(OCH₂CH₂)ₘ-OCOCH=CH₂; CF₃-O-(CH₂)₂₃-(OCH₂CH₂)ₘ-OCH=CH₂; CF₃-O-(CH₂)₂₃-(OCH₂CH₂)ₘ-OAr(NCO)ₚ |
| CF₃-O-(CH₂)₂₄-(OCH₂CH₂)ₘ-OCOCH=CH₂; CF₃-O-(CH₂)₂₄-(OCH₂CH₂)ₘ-OCH=CH₂; CF₃-O-(CH₂)₂₄-(OCH₂CH₂)ₘ-OAr(NCO)ₚ |
| SF₅-(CH₂)₄-(OCH₂CH₂)ₘ-OCOCH=CH₂; SF₅-(CH₂)₄-(OCH₂CH₂)ₘ-OCH=CH₂; SF₅-(CH₂)₄-(OCH₂CH₂)ₘ-OAr(NCO)ₚ |
| SF₅-(CH₂)₅-(OCH₂CH₂)ₘ-OCOCH=CH₂; SF₅-(CH₂)₅-(OCH₂CH₂)ₘ-OCH=CH₂; SF₅-(CH₂)₅-(OCH₂CH₂)ₘ-OAr(NCO)ₚ |
| SF₅-(CH₂)₆-(OCH₂CH₂)ₘ-OCOCH=CH₂; SF₅-(CH₂)₆-(OCH₂CH₂)ₘ-OCH=CH₂; SF₅-(CH₂)₆-(OCH₂CH₂)ₘ-OAr(NCO)ₚ |
| SF₅-(CH₂)₇-(OCH₂CH₂)ₘ-OCOCH=CH₂; SF₅-(CH₂)₇-(OCH₂CH₂)ₘ-OCH=CH₂; SF₅-(CH₂)₇-(OCH₂CH₂)ₘ-OAr(NCO)ₚ |
| SF₅-(CH₂)₈-(OCH₂CH₂)ₘ-OCOCH=CH₂; SF₅-(CH₂)₈-(OCH₂CH₂)ₘ-OCH=CH₂; SF₅-(CH₂)₈-(OCH₂CH₂)ₘ-OAr(NCO)ₚ |
| SF₅-(CH₂)₉-(OCH₂CH₂)ₘ-OCOCH=CH₂; SF₅-(CH₂)₉-(OCH₂CH₂)ₘ-OCH=CH₂; SF₅-(CH₂)₉-(OCH₂CH₂)ₘ-OAr(NCO)ₚ |
| SF₅-(CH₂)₁₀-(OCH₂CH₂)ₘ-OCOCH=CH₂; SF₅-(CH₂)₁₀-(OCH₂CH₂)ₘ-OCH=CH₂; SF₅-(CH₂)₁₀-(OCH₂CH₂)ₘ-OAr(NCO)ₚ |
| SF₅-(CH₂)₁₁-(OCH₂CH₂)ₘ-OCOCH=CH₂; SF₅-(CH₂)₁₁-(OCH₂CH₂)ₘ-OCH=CH₂; SF₅-(CH₂)₁₁-(OCH₂CH₂)ₘ-OAr(NCO)ₚ |
| SF₅-(CH₂)₁₂-(OCH₂CH₂)ₘ-OCOCH=CH₂; SF₅-(CH₂)₁₂-(OCH₂CH₂)ₘ-OCH=CH₂; SF₅-(CH₂)₁₂-(OCH₂CH₂)ₘ-OAr(NCO)ₚ |
| SF₅-(CH₂)₁₃-(OCH₂CH₂)ₘ-OCOCH=CH₂; SF₅-(CH₂)₁₃-(OCH₂CH₂)ₘ-OCH=CH₂; SF₅-(CH₂)₁₃-(OCH₂CH₂)ₘ-OAr(NCO)ₚ |
| SF₅-(CH₂)₁₄-(OCH₂CH₂)ₘ-OCOCH=CH₂; SF₅-(CH₂)₁₄-(OCH₂CH₂)ₘ-OCH=CH₂; SF₅-(CH₂)₁₄-(OCH₂CH₂)ₘ-OAr(NCO)ₚ |
| SF₅-(CH₂)₁₅-(OCH₂CH₂)ₘ-OCOCH=CH₂; SF₅-(CH₂)₁₅-(OCH₂CH₂)ₘ-OCH=CH₂; SF₅-(CH₂)₁₅-(OCH₂CH₂)ₘ-OAr(NCO)ₚ |
| SF₅-(CH₂)₁₆-(OCH₂CH₂)ₘ-OCOCH=CH₂; SF₅-(CH₂)₁₆-(OCH₂CH₂)ₘ-OCH=CH₂; SF₅-(CH₂)₁₆-(OCH₂CH₂)ₘ-OAr(NCO)ₚ |
| SF₅-(CH₂)₁₇-(OCH₂CH₂)ₘ-OCOCH=CH₂; SF₅-(CH₂)₁₇-(OCH₂CH₂)ₘ-OCH=CH₂; SF₅-(CH₂)₁₇-(OCH₂CH₂)ₘ-OAr(NCO)ₚ |
| SF₅-(CH₂)₁₈-(OCH₂CH₂)ₘ-OCOCH=CH₂; SF₅-(CH₂)₁₈-(OCH₂CH₂)ₘ-OCH=CH₂; SF₅-(CH₂)₁₈-(OCH₂CH₂)ₘ-OAr(NCO)ₚ |
| SF₅-(CH₂)₁₉-(OCH₂CH₂)ₘ-OCOCH=CH₂; SF₅-(CH₂)₁₉-(OCH₂CH₂)ₘ-OCH=CH₂; SF₅-(CH₂)₁₉-(OCH₂CH₂)ₘ-OAr(NCO)ₚ |
| SF₅-(CH₂)₂₀-(OCH₂CH₂)ₘ-OCOCH=CH₂; SF₅-(CH₂)₂₀-(OCH₂CH₂)ₘ-OCH=CH₂; SF₅-(CH₂)₂₀-(OCH₂CH₂)ₘ-OAr(NCO)ₚ |
| SF₅-(CH₂)₂₁-(OCH₂CH₂)ₘ-OCOCH=CH₂; SF₅-(CH₂)₂₁-(OCH₂CH₂)ₘ-OCH=CH₂; SF₅-(CH₂)₂₁-(OCH₂CH₂)ₘ-OAr(NCO)ₚ |
| SF₅-(CH₂)₂₂-(OCH₂CH₂)ₘ-OCOCH=CH₂; SF₅-(CH₂)₂₂-(OCH₂CH₂)ₘ-OCH=CH₂; SF₅-(CH₂)₂₂-(OCH₂CH₂)ₘ-OAr(NCO)ₚ |
| SF₅-(CH₂)₂₃-(OCH₂CH₂)ₘ-OCOCH=CH₂; SF₅-(CH₂)₂₃-(OCH₂CH₂)ₘ-OCH=CH₂; SF₅-(CH₂)₂₃-(OCH₂CH₂)ₘ-OAr(NCO)ₚ |
| SF₅-(CH₂)₂₄-(OCH₂CH₂)ₘ-OCOCH=CH₂; SF₅-(CH₂)₂₄-(OCH₂CH₂)ₘ-OCH=CH₂; SF₅-(CH₂)₂₄-(OCH₂CH₂)ₘ-OAr(NCO)ₚ |
| SF₅-CH=CH-(CH₂)₄-(OCH₂CH₂)ₘ-OCOCH=CH₂; SF₅-CH=CH-(CH₂)₄-(OCH₂CH₂)ₘ-OCH=CH₂; SF₅-CH=CH-(CH₂)₄-(OCH₂CH₂)ₘ-OAr(NCO)ₚ |
| SF₅-CH=CH-(CH₂)₅-(OCH₂CH₂)ₘ-OCOCH=CH₂; SF₅-CH=CH-(CH₂)₅-(OCH₂CH₂)ₘ-OCH=CH₂; SF₅-CH=CH-(CH₂)₅-(OCH₂CH₂)ₘ-OAr(NCO)ₚ |
| SF₅-CH=CH-(CH₂)₆-(OCH₂CH₂)ₘ-OCOCH=CH₂; SF₅-CH=CH-(CH₂)₆-(OCH₂CH₂)ₘ-OCH=CH₂, SF₅-CH=CH-(CH₂)₆-(OCH₂CH₂)ₘ-OAr(NCO)ₚ |
| SF₅-CH=CH-(CH₂)₇-(OCH₂CH₂)ₘ-OCOCH=CH₂; SF₅-CH=CH-(CH₂)₇-(OCH₂CH₂)ₘ-OCH=CH₂; SF₅-CH=CH-(CH₂)₇-(OCH₂CH₂)ₘ-OAr(NCO)ₚ |
| SF₅-CH=CH-(CH₂)₈-(OCH₂CH₂)ₘ-OCOCH=CH₂; SF₅-CH=CH-(CH₂)₈-(OCH₂CH₂)ₘ-OCH=CH₂; SF₅-CH=CH-(CH₂)₈-(OCH₂CH₂)ₘ-OAr(NCO)ₚ |
| SF₅-CH=CH-(CH₂)₉-(OCH₂CH₂)ₘ-OCOCH=CH₂; SF₅-CH=CH-(CH₂)₉-(OCH₂CH₂)ₘ-OCH=CH₂; SF₅-CH=CH-(CH₂)₉-(OCH₂CH₂)ₘ-OAr(NCO)ₚ |
| SF₅-CH=CH-(CH₂)₁₀-(OCH₂CH₂)ₘ-OCOCH=CH₂; SF₅-CH=CH-(CH₂)₁₀-(OCH₂CH₂)ₘ-OCH=CH₂; SF₅-CH=CH-(CH₂)₁₀-(OCH₂CH₂)ₘ-OAr(NCO)ₚ |
| SF₅-CH=CH-(CH₂)₁₁-(OCH₂CH₂)ₘ-OCOCH=CH₂; SF₅-CH=CH-(CH₂)₁₁-(OCH₂CH₂)ₘ-OCH=CH₂; SF₅-CH=CH-(CH₂)₁₁-(OCH₂CH₂)ₘ-OAr(NCO)ₚ |
| SF₅-CH=CH-(CH₂)₁₂-(OCH₂CH₂)ₘ-OCOCH=CH₂; SF₅-CH=CH-(CH₂)₁₂-(OCH₂CH₂)ₘ-OCH=CH₂; SF₅-CH=CH-(CH₂)₁₂-(OCH₂CH₂)ₘ-OAr(NCO)ₚ |
| SF₅-CH=CH-(CH₂)₁₃-(OCH₂CH₂)ₘ-OCOCH=CH₂; SF₅-CH=CH-(CH₂)₁₃-(OCH₂CH₂)ₘ-OCH=CH₂; SF₅-CH=CH-(CH₂)₁₃-(OCH₂CH₂)ₘ-OAr(NCO)ₚ |
| SF₅-CH=CH-(CH₂)₁₄-(OCH₂CH₂)ₘ-OCOCH=CH₂; SF₅-CH=CH-(CH₂)₁₄-(OCH₂CH₂)ₘ-OCH=CH_{2;} SF₅-CH=CH-(CH₂)₁₄-(OCH₂CH₂)ₘ-OAr(NCO)ₚ |
| SF₅-CH=CH-(CH₂)₁₅-(OCH₂CH₂)ₘ-OCOCH=CH₂; SF₅-CH=CH-(CH₂)₁₅-(OCH₂CH₂)ₘ-OCH=CH₂; SF₅-CH=CH-(CH₂)₁₅-(OCH₂CH₂)ₘ-OAr(NCO)ₚ |
| SF₅-CH=CH-(CH₂)₁₆-(OCH₂CH₂)ₘ-OCOCH=CH₂; SF₅-CH=CH-(CH₂)₁₆-(OCH₂CH₂)ₘ-OCH=CH₂; SF₅-CH=CH-(CH₂)₁₆-(OCH₂CH₂)ₘ-OAr(NCO)ₚ |
| SF₅-CH=CH-(CH₂)₁₇-(OCH₂CH₂)ₘ-OCOCH=CH₂; SF₅-CH=CH-(CH₂)₁₇-(OCH₂CH₂)ₘ-OCH=CH₂; SF₅-CH=CH-(CH₂)₁₇-(OCH₂CH₂)ₘ-OAr(NCO)ₚ |
| SF₅-CH=CH-(CH₂)₁₈-(OCH₂CH₂)ₘ-OCOCH=CH₂; SF₅-CH=CH-(CH₂)₁₈-(OCH₂CH₂)ₘ-OCH=CH₂; SF₅-CH=CH-(CH₂)₁₈-(OCH₂CH₂)ₘ-OAr(NCO)ₚ |
| SF₅-CH=CH-(CH₂)₁₉-(OCH₂CH₂)ₘ-OCOCH=CH₂; SF₅-CH=CH-(CH₂)₁₉-(OCH₂CH₂)ₘ-OCH=CH₂; SF₅-CH=CH-(CH₂)₁₉-(OCH₂CH₂)ₘ-OAr(NCO)ₚ |
| SF₅-CH=CH-(CH₂)₂₀-(OCH₂CH₂)ₘ-OCOCH=CH₂; SF₅-CH=CH-(CH₂)₂₀-(OCH₂CH₂)ₘ-OCH=CH₂; SF₅-CH=CH-(CH₂)₂₀-(OCH₂CH₂)ₘ-OAr(NCO)ₚ |
| SF₅-CH=CH-(CH₂)₂₁-(OCH₂CH₂)ₘ-OCOCH=CH₂; SF₅-CH=CH-(CH₂)₂₁-(OCH₂CH₂)ₘ-OCH=CH₂; SF₅-CH=CH-(CH₂)₂₁-(OCH₂CH₂)ₘ-OAr(NCO)ₚ |
| SF₅-CH=CH-(CH₂)₂₂-(OCH₂CH₂)ₘ-OCOCH=CH₂; SF₅-CH=CH-(CH₂)₂₂-(OCH₂CH₂)ₘ-OCH=CH₂; SF₅-CH=CH-(CH₂)₂₂-(OCH₂CH₂)ₘ-OAr(NCO)ₚ |
| SF₅-CH=CH-(CH₂)₂₃-(OCH₂CH₂)ₘ-OCOCH=CH₂; SF₅-CH=CH-(CH₂)₂₃-(OCH₂CH₂)ₘ-OCH=CH₂; SF₅-CH=CH-(CH₂)₂₃-(OCH₂CH₂)ₘ-OAr(NCO)ₚ |
| SF₅-CH=CH-(CH₂)₂₄-(OCH₂CH₂)ₘ-OCOCH=CH₂; SF₅-CH=CH-(CH₂)₂₄-(OCH₂CH₂)ₘ-OCH=CH₂; SF₅-CH=CH-(CH₂)₂₄-(OCH₂CH₂)ₘ-OAr(NCO)ₚ |
| SF₅-CH₂CHBr-(CH₂)₄-(OCH₂CH₂)ₘ-OCOCH=CH₂; SF₅-CH₂CHBr-(CH₂)₄-(OCH₂CH₂)ₘ-OCH=CH₂; SF₅-CH₂CHBr-(CH₂)₄-(OCH₂CH₂)ₘ-OAr(NCO)ₚ |
| SF₅-CH₂CHBr-(CH₂)₅-(OCH₂CH₂)ₘ-OCOCH=CH₂; SF₅-CH₂CHBr-(CH₂)₅-(OCH₂CH₂)ₘ-OCH=CH₂; SF₅-CH₂CHBr-(CH₂)₅-(OCH₂CH₂)ₘ-OAr(NCO)ₚ |
| SF₅-CH₂CHBr-(CH₂)₆-(OCH₂CH₂)ₘ-OCOCH=CH₂; SF₅-CH₂CHBr-(CH₂)₆-(OCH₂CH₂)ₘ-OCH=CH₂; SF₅-CH₂CHBr-(CH₂)₆-(OCH₂CH₂)ₘ-OAr(NCO)ₚ |
| SF₅-CH₂CHBr-(CH₂)₇-(OCH₂CH₂)ₘ-OCOCH=CH₂; SF₅-CH₂CHBr-(CH₂)₇-(OCH₂CH₂)ₘ=OCH=CH₂; SF₅-CH₂CHBr-(CH₂)₇-(OCH₂CH₂)ₘ-OAr(NCO)ₚ |
| SF₅-CH₂CHBr-(CH₂)₈-(OCH₂CH₂)ₘ-OCOCH=CH₂; SF₅-CH₂CHBr-(CH₂)₈-(OCH₂CH₂)ₘ-OCH=CH₂; SF₅-CH₂CHBr-(CH₂)₈-(OCH₂CH₂)ₘ-OAr(NCO)ₚ |
| SF₅-CH₂CHBr-(CH₂)₈-(OCH₂CH₂)ₘ-OCOCH=CH₂; SF₅-CH₂CHBr-(CH₂)₉-(OCH₂CH₂)ₘ-OCH=CH₂; SF₅-CH₂CHBr-(CH₂)₉-(OCH₂CH₂)ₘ-OAr(NCO)ₚ |
| SF₅-CH₂CHBr-(CH₂)₁₀-(OCH₂CH₂)ₘ-OCOCH=CH₂; SF₅-CH₂CHBr-(CH₂)₁₀-(OCH₂CH₂)ₘ-OCH=CH₂; SF₅-CH₂CHBr-(CH₂)₁₀-(OCH₂CH₂)ₘ-OAr(NCO)ₚ |
| SF₅-CH₂CHBr-(CH₂)₁₁-(OCH₂CH₂)ₘ-OCOCH=CH₂; SF₅-CH₂CHBr-(CH₂)₁₁-(OCH₂CH₂)ₘ-OCH=CH₂; SF₅-CH₂CHBr-(CH₂)₁₁-(OCH₂CH₂)ₘ-OAr(NCO)ₚ |
| SF₅-CH₂CHBr-(CH₂)₁₂-(OCH₂CH₂)ₘ-OCOCH=CH₂; SF₅-CH₂CHBr-(CH₂)₁₂-(OCH₂CH₂)ₘ-OCH=CH₂; SF₅-CH₂CHBr-(CH₂)₁₂-(OCH₂CH₂)ₘ-OAr(NCO)ₚ |
| SF₅-CH₂CHBr-(CH₂)₁₃-(OCH₂CH₂)ₘ-OCOCH=CH₂; SF₅-CH₂CHBr-(CH₂)₁₃-(OCH₂CH₂)ₘ-OCH=CH₂; SF₅-CH₂CHBr-(CH₂)₁₃-(OCH₂CH₂)ₘ-OAr(NCO)ₚ |
| SF₅-CH₂CHBr-(CH₂)₁₄-(OCH₂CH₂)ₘ-OCOCH=CH₂; SF₅-CH₂CHBr-(CH₂)₁₄-(OCH₂CH₂)ₘ-OCH=CH₂; SF₅-CH₂CHBr-(CH₂)₁₄-(OCH₂CH₂)ₘ-OAr(NCO)ₚ |
| SF₅-CH₂CHBr-(CH₂)₁₅-(OCH₂CH₂)ₘ-OCOCH=CH₂; SF₅CH₂CHBr-(CH₂)₁₅-(OCH₂CH₂)ₘ-OCH=CH₂; SF₅-CH₂CHBr-(CH₂)₁₅-(OCH₂CH₂)ₘ-OAr(NCO)ₚ |
| SF₅-CH₂CHBr-(CH₂)₁₆-(OCH₂CH₂)ₘ-OCOCH=CH₂; SF₅-CH₂CHBr-(CH₂)₁₆-(OCH₂CH₂)ₘ-OCH=CH₂; SF₅-CH₂CHBr-(CH₂)₁₆-(OCH₂CH₂)ₘ-OAr(NCO)ₚ |
| SF₅-CH₂CHBr-(CH₂)₁₇-(OCH₂CH₂)ₘ-OCOCH=CH₂; SF₅-CH₂CHBr-(CH₂)₁₇-(OCH₂CH₂)ₘ-OCH=CH₂; SF₅-CH₂CHBr-(CH₂)₁₇-(OCH₂CH₂)ₘ-OAr(NCO)ₚ |
| SF₅-CH₂CHBr-(CH₂)₁₈-(OCH₂CH₂)ₘ-OCOCH=CH₂; SF₅-CH₂CHBr-(CH₂)₁₈-(OCH₂CH₂)ₘ-OCH=CH₂; SF₅-CH₂CHBr-(CH₂)₁₈-(OCH₂CH₂)ₘ-OAr(NCO)ₚ |
| SF₅-CH₂CHBr-(CH₂)₁₉-(OCH₂CH₂)ₘ-OCOCH=CH₂; SF₅-CH₂CHBr-(CH₂)₁₉-(OCH₂CH₂)ₘ-OCH=CH₂; SF₅-CH₂CHBr-(CH₂)₁₉-(OCH₂CH₂)ₘ-OAr(NCO)ₚ |
| SF₅-CH₂CHBr-(CH₂)₂₀-(OCH₂CH₂)ₘ-OCOCH=CH₂; SF₅-CH₂CHBr-(CH₂)₂₀-(OCH₂CH₂)ₘ-OCH=CH₂; SF₅-CH₂CHBr-(CH₂)₂₀-(OCH₂CH₂)ₘ-OAr(NCO)ₚ |
| SF₅-CH₂CHBr-(CH₂)₂₁-(OCH₂CH₂)ₘ-OCOCH=CH₂; SF₅-CH₂CHBr-(CH₂)₂₁-(OCH₂CH₂)ₘ-OCH=CH₂; SF₅-CH₂CHBr-(CH₂)₂₁-(OCH₂CH₂)ₘ-OAr(NCO)ₚ |
| SF₅-CH₂CHBr-(CH₂)₂₂-(OCH₂CH₂)ₘ-OCOCH=CH₂; SF₅-CH₂CHBr-(CH₂)₂₂-(OCH₂CH₂)ₘ-OCH=CH₂; SF₅-CH₂CHBr-(CH₂)₂₂-(OCH₂CH₂)ₘ-OAr(NCO)ₚ |
| SF₅-CH₂CHBr-(CH₂)₂₃-(OCH₂CH₂)ₘ-OCOCH=CH₂; SF₅-CH₂CHBr-(CH₂)₂₃-(OCH₂CH₂)ₘ-OCH=CH₂; SF₅-CH₂CHBr-(CH₂)₂₃-(OCH₂CH₂)ₘ-OAr(NCO)ₚ |
| SF₅-CH₂CHBr-(CH₂)₂₄-(OCH₂CH₂)ₘ-OCOCH=CH₂; SF₅-CH₂CHBr-(CH₂)₂₄-(OCH₂CH₂)ₘ-OCH=CH₂; SF₅-CH₂CHBr-(CH₂)₂₄-(OCH₂CH₂)ₘ-OAr(NCO)ₚ |
| CF₃-O-(CH₂)₄-N⁺R¹R²R³Z⁻; CF₃-O-(CH₂)₄-P⁺R¹R²R³Z⁻; CF₃-O-(CH₂)₄-O-Glucosid |
| CF₃-O-(CH₂)₅-N⁺R¹R²R³Z⁻; CF₃-O-(CH₂)₅-P⁺R¹R²R³Z⁻; CF₃-O-(CH₂)₅-O-Glucosid |
| CF₃-O-(CH₂)₆-N⁺R¹R²R³Z⁻; CF₃-O-(CH₂)₆-P⁺R¹R²R³Z⁻; CF₃-O-(CH₂)₆-O-Glucosid |
| CF₃-O-(CH₂)₇-N⁺R¹R²R³Z⁻; CF₃-O-(CH₂)₇-P⁺R¹R²R³Z⁻; CF₃-O-(CH₂)₇-O-Glucosid |
| CF₃-O-(CH₂)₈-N⁺R¹R²R³Z⁻; CF₃-O-(CH₂)₈-P⁺R¹R²R³Z⁻; CF₃-O-(CH₂)₈-O-Glucosid |
| CF₃-O-(CH₂)₉-N⁺R¹R²R³Z⁻; CF₃-O-(CH₂)₉-P⁺R¹R²R³Z⁻; CF₃-O-(CH₂)₉-O-Glucosid |
| CF₃-O-(CH₂)₁₀-N⁺R¹R²R³Z⁻; CF₃-O-(CH₂)₁₀-P⁺R¹R²R³Z⁻; CF₃-O-(CH₂)₁₀-O-Glucosid |
| CF₃-O-(CH₂)₁₁-N⁺R¹R²R³Z⁻; CF₃-O-(CH₂)₁₁-P⁺R¹R²R³Z⁻; CF₃-O-(CH₂)₁₁-O-Glucosid |
| CF₃-O-(CH₂)₁₂-N⁺R¹R²R³Z⁻; CF₃-O-(CH₂)₁₂-P⁺R¹R²R³Z⁻; CF₃-O-(CH₂)₁₂-O-Glucosid |
| CF₃-O-(CH₂)₁₃-N⁺R¹R²R³Z⁻; CF₃-O-(CH₂)₁₃-P⁺R¹R²R³Z⁻; CF₃-O-(CH₂)₁₃-O-Glucosid |
| CF₃-O-(CH₂)₁₄-N⁺R¹R²R³Z⁻; CF₃-O-(CH₂)₁₄-P⁺R¹R²R³Z⁻; CF₃-O-(CH₂)₁₄-O-Glucosid |
| CF₃-O-(CH₂)₁₅-N⁺R¹R²R³Z⁻; CF₃-O-(CH₂)₁₅-P⁺R¹R²R³Z⁻; CF₃-O-(CH₂)₁₅-O-Glucosid |
| CF₃-O-(CH₂)₁₆-N⁺R¹R²R³Z⁻; CF₃-O-(CH₂)₁₆-P⁺R¹R²R³Z⁻; CF₃-O-(CH₂)₁₆-O-Glucosid |
| CF₃-O-(CH₂)₁₇-N⁺R¹R²R³Z⁻; CF₃-O-(CH₂)₁₇-P⁺R¹R²R³Z⁻; CF₃-O-(CH₂)₁₇-O-Glucosid |
| CF₃-O-(CH₂)₁₈-N⁺R¹R²R³Z⁻; CF₃-O-(CH₂)₁₈-P⁺R¹R²R³Z⁻; CF₃-O-(CH₂)₁₈-O-Glucosid |
| CF₃-O-(CH₂)₁₉-N⁺R¹R²R³Z⁻; CF₃-O-(CH₂)₁₉-P⁺R¹R²R³Z⁻; CF₃-O-(CH₂)₁₉-O-Glucosid |
| CF₃-O-(CH₂)₂₀-N⁺R¹R²R³Z⁻; CF₃-O-(CH₂)₂₀-P⁺R¹R²R³Z⁻; CF₃-O-(CH₂)₂₀-O-Glucosid |
| CF₃-O-(CH₂)₂₁-N⁺R¹R²R³Z⁻; CF₃-O-(CH₂)₂₁-P⁺R¹R²R³Z⁻; CF₃-O-(CH₂)₂₁-O-Glucosid |
| CF₃-O-(CH₂)₂₂-N⁺R¹R²R³Z⁻; CF₃-O-(CH₂)₂₂-P⁺R¹R²R³Z⁻; CF₃-O-(CH₂)₂₂-O-Glucosid |
| CF₃-O-(CH₂)₂₃-N⁺R¹R²R³Z⁻; CF₃-O-(CH₂)₂₃-P⁺R¹R²R³Z⁻; CF₃-O-(CH₂)₂₃-O-Glucosid |
| CF₃-O-(CH₂)₂₄-N⁺R¹R²R³Z⁻; CF₃-O-(CH₂)₂₄-P⁺R¹R²R³Z⁻; CF₃-O-(CH₂)₂₄-O-Glucosid |
| SF₅-(CH₂)₄-N⁺R¹R²R³Z⁻; SF₅-(CH₂)₄-P⁺R¹R²R³Z⁻; SF₅-(CH₂)₄-O-Glucosid |
| SF₅-(CH₂)₅-N⁺R¹R²R³Z⁻; SF₅-(CH₂)₅-P⁺R¹R²R³Z⁻; SF₆-(CH₂)₅-O-Glucosid |
| SF₅-(CH₂)6-N⁺R¹R²R³Z⁻; SF₅-(CH₂)₆-P⁺R¹R²R³Z⁻; SF₅-(CH₂)₆-O-Glucosid |
| SF₅-(CH₂)₇-N⁺R¹R²R³Z⁻; SF₅-(CH₂)₇-P⁺R¹R²R³Z⁻; SF₅-(CH₂)₇-O-Glucosid |
| SF₅-(CH₂)₈-N⁺R¹R²R³Z⁻; SF₅-(CH₂)₈-P⁺R¹R²R³Z⁻; SF₅-(CH₂)₈-O-Glucosid |
| SF₅-(CH₂)₉-N⁺R¹R²R³Z⁻; SF₅-(CH₂)₉-P⁺R¹R²R³Z⁻; SF₅-(CH₂)₉-O-Glucosid |
| SF₅-(CH₂)₁₀-N⁺R¹R²R³Z⁻; SF₅-(CH₂)₁₀-P⁺R¹R²R³Z⁻, SF₅-(CH₂)₁₀-O-Glucosid |
| SF₅-(CH₂)₁₁-N⁺R¹R²R³Z⁻; SF₅-(CH₂)₁₁-P⁺R¹R²R³Z⁻; SF₅-(CH₂)₁₁-O-Glucosid |
| SF₅-(CH₂)₁₂-N⁺R¹R²R³Z⁻; SF₅-(CH₂)₁₂-P⁺R¹R²R³Z⁻; SF₅-(CH₂)₁₂-O-Glucosid |
| SF₅-(CH₂)₁₃-N⁺R¹R²R³Z⁻; SF₅-(CH₂)₁₃-P⁺R¹R²R³Z⁻; SF₅-(CH₂)₁₃-O-Glucosid |
| SF₅-(CH₂)₁₄-N⁺R¹R²R³Z⁻; SF₅-(CH₂)₁₄-P⁺R¹R²R³Z⁻; SF₅-(CH₂)₁₄-O-Glucosid |
| SF₅-(CH₂)₁₅-N⁺R¹R²R³Z⁻; SF₅-(CH₂)₁₅-P⁺R¹R²R³Z⁻; SF₅-(CH₂)₁₅-O-Glucosid |
| SF₅-(CH₂)₁₆-N⁺R¹R²R³Z⁻; SF₅-(CH₂)₁₆-P⁺R¹R²R³Z⁻; SF₅-(CH₂)₁₆-O-Glucosid |
| SF₅-(CH₂)₁₇-N⁺R¹R²R³Z⁻; SF₅-(CH₂)₁₇-P⁺R¹R²R³Z⁻; SF₅-(CH₂)₁₇-O-Glucosid |
| SF₅-(CH₂)₁₈-N⁺R¹R²R³Z⁻; SF₅-(CH₂)₁₈-P⁺R¹R²R³Z⁻; SF₅-(CH₂)₁₈-O-Glucosid |
| SF₅-(CH₂)₁₉-N⁺R¹R²R³Z⁻; SF₅-(CH₂)₁₉-P⁺R¹R²R³Z⁻; SF₅-(CH₂)₁₉-O-Glucosid |
| SF₅-(CH₂)₂₀-N⁺R¹R²R³Z⁻; SF₅-(CH₂)₂₀-P⁺R¹R²R³Z⁻; SF₅-(CH₂)₂₀-O-Glucosid |
| SF₅-(CH₂)₂₁-N⁺R¹R²R³Z⁻; SF₅-(CH₂)₂₁-P⁺R¹R²R³Z⁻; SF₅-(CH₂)₂₁-O-Glucosid |
| SF₅-(CH₂)₂₂-N⁺R¹R²R³Z⁻; SF₅-(CH₂)₂₂-P⁺R¹R²R³Z⁻; SF₅-(CH₂)₂₂-O-Glucosid |
| SF₅-(CH₂)₂₃-N⁺R¹R²R³Z⁻; SF₅-(CH₂)₂₃-P⁺R¹R²R³Z⁻; SF₅-(CH₂)₂₃-O-Glucosid |
| SF₅-(CH₂)₂₄-N⁺R¹R²R³Z⁻; SF₅-(CH₂)₂₄-P⁺R¹R²R³Z⁻; SF₅-(CH₂)₂₄-O-Glucosid |
| SF₅-CH=CH-(CH₂)₄-N⁺R¹R²R³Z⁻; SF₅-CH=CH-(CH₂)₄-P⁺R¹R²R³Z⁻; SF₅-CH=CH-(CH₂)₄-O-Glucosid |
| SF₅-CH=CH-(CH₂)₅-N⁺R¹R²R³Z⁻; SF₅-CH=CH-(CH₂)₅-P⁺R¹R²R³Z⁻; SF₅-CH=CH-(CH₂)₅-O-Glucosid |
| SF₅-CH=CH-(CH₂)₆-N⁺R¹R²R³Z⁻; SF₅-CH=CH-(CH₂)₆-P⁺R¹R²R³Z⁻; SF₅-CH=CH-(CH₂)₆-O-Glucosid |
| SF₅-CH=CH-(CH₂)₇-N⁺R¹R²R³Z⁻; SF₅-CH=CH-(CH₂)₇-P⁺R¹R²R³Z⁻; SF₅-CH=CH-(CH₂)₇-O-Glucosid |
| SF₅-CH=CH-(CH₂)₈-N⁺R¹R²R³Z⁻; SF₅-CH=CH-(CH₂)₈-P⁺R¹R²R³Z⁻; SF₅-CH=CH-(CH₂)₈-O-Glucosid |
| SF₅-CH=CH-(CH₂)₉-N⁺R¹R²R³Z⁻; SF₅-CH=CH-(CH₂)9-P⁺R¹R²R³Z⁻; SF₅-CH=CH-(CH₂)₉-O-Glucosid |
| SF₅-CH=CH-(CH₂)₁₀-N⁺R¹R²R³Z⁻; SF₅-CH=CH-(CH₂)₁₀-P⁺R¹R²R³Z⁻; SF₅-CH=CH-(CH₂)₁₀-O-Glucosid |
| SF₅-CH=CH-(CH₂)₁₁-N⁺R¹R²R³Z⁻; SF₅-CH=CH-(CH₂)₁₁-P⁺R¹R²R³Z⁻; SF₅-CH=CH-(CH₂)₁₁-O-Glucosid |
| SF₅-CH=CH-(CH₂)₁₂-N⁺R¹R²R³Z⁻; SF₅-CH=CH-(CH₂)₁₂-P⁺R¹R²R³Z⁻; SF₅-CH=CH-(CH₂)₁₂-O-Glucosid |
| SF₅-CH=CH-(CH₂)₁₃-N⁺R¹R²R³Z⁻; SF₅-CH=CH-(CH₂)₁₃-P⁺R¹R²R³Z⁻; SF₅-CH=CH-(CH₂)₁₃-O-Glucosid |
| SF₅-CH=CH-(CH₂)₁₄-N⁺R¹R²R³Z⁻; SF₅-CH=CH-(CH₂)₁₄-P⁺R¹R²R³Z⁻; SF₅-CH=CH-(CH₂)₁₄-O-Glucosid |
| SF₅-CH=CH-(CH₂)₁₅-N⁺R¹R²R³Z⁻; SF₅-CH=CH-(CH₂)₁₅-P⁺R¹R²R³Z⁻; SF₅-CH=CH-(CH₂)₁₅-O-Glucosid |
| SF₅-CH=CH-(CH₂)₁₆-N⁺R¹R²R³Z⁻; SF₅-CH=CH-(CH₂)₁₆-P⁺R¹R²R³Z⁻; SF₅-CH=CH-(CH₂)₁₆-O-Glucosid |
| SF₅-CH=CH-(CH₂)₁₇-N⁺R¹R²R³Z⁻; SF₅-CH=CH-(CH₂)₁₇-P⁺R¹R²R³Z⁻; SF₅-CH=CH-(CH₂)₁₇-O-Glucosid |
| SF₅-CH=CH-(CH₂)₁₈-N⁺R¹R²R³Z⁻; SF₅-CH=CH-(CH₂)₁₈-P⁺R¹R²R³Z⁻; SF₅-CH=CH-(CH₂)₁₈-O-Glucosid |
| SF₅-CH=CH-(CH₂)₁₉-N⁺R¹R²R³Z⁻; SF₅-CH=CH-(CH₂)₁₉-P⁺R¹R²R³Z⁻; SF₅-CH=CH-(CH₂)₁₉-O-Glucosid |
| SF₅-CH=CH-(CH₂)₂₀-N⁺R¹R²R³Z⁻; SF₅-CH=CH-(CH₂)₂₀-P⁺R¹R²R³Z⁻; SF₅-CH=CH-(CH₂)₂₀-O-Glucosid |
| SF₅-CH=CH-(CH₂)₂₁-N⁺R¹R²R³Z⁻; SF₅-CH=CH-(CH₂)₂₁-P⁺R¹R²R³Z⁻; SF₅-CH=CH-(CH₂)₂₁-O-Glucosid |
| SF₅-CH=CH-(CH₂)₂₂-N⁺R¹R²R³Z⁻; SF₅-CH=CH-(CH₂)₂₂-P⁺R¹R²R³Z⁻; SF₅-CH=CH-(CH₂)₂₂-O-Glucosid |
| SF₅-CH=CH-(CH₂)₂₃-N⁺R¹R²R³Z⁻; SF₅-CH=CH-(CH₂)₂₃-P⁺R¹R²R³Z⁻; SF₅-CH=CH-(CH₂)₂₃-O-Glucosid |
| SF₅-CH=CH-(CH₂)₂₄-N⁺R¹R²R³Z⁻; SF₅-CH=CH-(CH₂)₂₄-P⁺R¹R²R³Z⁻; SF₅-CH=CH-(CH₂)₂₄-O-Glucosid |
| SF₅-CH₂CHBr-(CH₂)₄-N⁺R¹R²R³Z⁻; SF₅-CH₂CHBr-(CH₂)₄-P⁺R¹R²R³Z⁻; SF₅-CH₂CHBr-(CH₂)₄-O-Glucosid |
| SF₅-CH₂CHBr-(CH₂)₅-N⁺R¹R²R³Z⁻; SF₅-CH₂CHBr-(CH₂)₅-P⁺R¹R²R³Z⁻; SF₅-CH₂CHBr-(CH₂)₅-O-Glucosid |
| SF₅-CH₂CHBr-(CH₂)₆-N⁺R¹R²R³Z⁻; SF₅-CH₂CHBr-(CH₂)₆-P⁺R¹R²R³Z⁻; SF₅-CH₂CHBr-(CH₂)₆-O-Glucosid |
| SF₅-CH₂CHBr-(CH₂)₇-N⁺R¹R²R³Z⁻; SF₅-CH₂CHBr-(CH₂)₇-P⁺R¹R²R³Z⁻; SF₅-CH₂CHBr-(CH₂)₇-O-Glucosid |
| SF₅-CH₂CHBr-(CH₂)₈-N⁺R¹R²R³Z⁻; SF₅-CH₂CHBr-(CH₂)₈-P⁺R¹R²R³Z⁻; SF₅-CH₂CHBr-(CH₂)₈-O-Glucosid |
| SF₅-CH₂CHBr-(CH₂)9-N⁺R¹R²R³Z⁻; SF₅-CH₂CHBr-(CH₂)₉-P⁺R¹R²R³Z⁻; SF₅-CH₂CHBr-(CH₂)₉-O-Glucosid |
| SF₅-CH₂CHBr-(CH₂)₁₀-N⁺R¹R²R³Z⁻; SF₅-CH₂CHBr-(CH₂)₁₀-P⁺R¹R²R³Z⁻; SF₅-CH₂CHBr-(CH₂)₁₀-O-Glucosid |
| SF₅-CH₂CHBr-(CH₂)₁₁-N⁺R¹R²R³Z⁻; SF₅-CH₂CHBr-(CH₂)₁₂-P⁺R¹R²R³Z⁻; SF₅-CH₂CHBr-(CH₂)₁₁-O-Glucosid |
| SF₅-CH₂CHBr-(CH₂)₁₂-N⁺R¹R²R³Z⁻; SF₅-CH₂CHBr-(CH₂)₁₂-P⁺R¹R²R³Z⁻; SF₅-CH₂CHBr-(CH₂)₁₂-O-Glucosid |
| SF₅-CH₂CHBr-(CH₂)₁₃-N⁺R¹R²R³Z⁻; SF₅-CH₂CHBr-(CH₂)₁₃-P⁺R¹R²R³Z⁻; SF₅-CH₂CHBr-(CH₂)₁₃-O-Glucosid |
| SF₅-CH₂CHBr-(CH₂)₁₄-N⁺R¹R²R³Z⁻; SF₅-CH₂CHBr-(CH₂)₁₄-P⁺R¹R²R³Z⁻; SF₅-CH₂CHBr-(CH₂)₁₄-O-Glucosid |
| SF₅-CH₂CHBr-(CH₂)₁₅-N⁺R¹R²R³Z⁻; SF₅-CH₂CHBr-(CH₂)₁₅-P⁺R¹R²R³Z⁻; SF₅-CH₂CHBr-(CH₂)₁₅-O-Glucosid |
| SF₅-CH₂CHBr-(CH₂)₁₆-N⁺R¹R²R³Z⁻; SF₅-CH₂CHBr-(CH₂)₁₆-P⁺R¹R²R³Z⁻; SF₅-CH₂CHBr-(CH₂)₁₆-O-Glucosid |
| SF₅-CH₂CHBr-(CH₂)₁₇-N⁺R¹R²R³Z⁻; SF₅-CH₂CHBr-(CH₂)₁₇-P⁺R¹R²R³Z⁻; SF₅-CH₂CHBr-(CH₂)₁₇-O-Glucosid |
| SF₅-CH₂CHBr-(CH₂)₁₈-N⁺R¹R²R³Z⁻; SF₅-CH₂CHBr-(CH₂)₁₈-P⁺R¹R²R³Z⁻; SF₅-CH₂CHBr-(CH₂)₁₈-O-Glucosid |
| SF₅-CH₂CHBr-(CH₂)₁₉-N⁺R¹R²R³Z⁻; SF₅-CH₂CHBr-(CH₂)₁₉-P⁺R¹R²R³Z⁻; SF₅-CH₂CHBr-(CH₂)₁₉-O-Glucosid |
| SF₅-CH₂CHBr-(CH₂)₂₀-N⁺R¹R²R³Z⁻; SF₅-CH₂CHBr-(CH₂)₂₀-P⁺R¹R²R³Z⁻; SF₅-CH₂CHBr-(CH₂)₂₀-O-Glucosid |
| SF₅-CH₂CHBr-(CH₂)₂₁-N⁺R¹R²R³Z⁻; SF₅-CH₂CHBr-(CH₂)₂₁-P⁺R¹R²R³Z⁻; SF₅-CH₂CHBr-(CH₂)₂₁-O-Glucosid |
| SF₅-CH₂CHBr-(CH₂)₂₂-N⁺R¹R²R³Z⁻; SF₅-CH₂CHBr-(CH₂)₂₂-P⁺R¹R²R³Z⁻; SF₅-CH₂CHBr-(CH₂)₂₂-O-Glucosid |
| SF₅-CH₂CHBr-(CH₂)₂₃-N⁺R¹R²R³Z⁻; SF₅-CH₂CHBr-(CH₂)₂₃-P⁺R¹R²R³Z⁻; SF₅-CH₂CHBr-(CH₂)₂₃-O-Glucosid |
| SF₅-CH₂CHBr-(CH₂)₂₄-N⁺R¹R²R³Z⁻; SF₅-CH₂CHBr-(CH₂)₂₄-P⁺R¹R²R³Z⁻; SF₅-CH₂CHBr-(CH₂)₂₄-O-Glucosid |

Dabei eignen sich die erfindungsgemäß als Tensid verwendbaren Verbindungen insbesondere zum Einsatz als Hydrophobiermittel, beispielsweise zur Oberflächenmodifikation von Textilien, Papier, Glas, poröser Baustoffe oder Adsorbentien, bzw. als Grenzflächenvermittler bzw. Emulgator, insbesondere für die Herstellung von Fluorpolymeren, bzw. als Viskositätsminderer oder Emulgator, insbesondere in Farben, Lacken oder Zubereitungen zur Oberflächenbeschichtung, bzw. als Schaumstabilisator, insbesondere in Zubereitungen, die als "Feuerlöschschäume" bekannt sind, bzw. in der Metallbearbeitung zum Abdecken galvanischer Bäder gegen den Austritt ätzender Dämpfe, bzw. als Netzmittel bei der Herstellung photographischer Filme und Papiere, bzw. als Verlaufsmittel in Selbstglanzemulsionen, bzw. als Feuerlöschmittel, sowie zur schmutzabweisenden Ausrüstung.

Darüber hinaus eignen sich die erfindungsgemäß als Tensid verwendbaren Verbindungen für Wasch- und Reinigungsanwendungen, insbesondere von Textilien. Auch Reinigen und Polieren harter Oberflächen ist ein mögliches Anwendungsgebiet für die erfindungsgemäß als Tensid verwendbaren Verbindungen. Weiter können die erfindungsgemäß als Tensid verwendbaren Verbindungen vorteilhaft in kosmetischen Produkten, wie beispielsweise Schaumbädern und Haarshampoos oder als Emulgatoren in Cremes und Lotionen eingesetzt werden. Ein weiteres Anwendungsgebiet für die erfindungsgemäß als Tensid verwendbaren Verbindungen ist die Flotation, d.h. das Ausbringen und Abtrennen von Erzen und Mineralien von taubem Gestein. Darüber hinaus können bevorzugte der erfindungsgemäß als Tensid verwendbaren Verbindungen auch als Emulgatoren in Nahrungsmitteln eingesetzt werden. Weitere Anwendungsfelder liegen in der Metallbehandlung, als Lederhilfsmittel, der Bauchemie und im Pflanzenschutz.

Weiter eignen sich erfindungsgemäße Tenside auch als antimikrobieller Wirkstoff, insbesondere als Reagentien für die antimikrobielle Oberflächenmodifikation. Von Vorteil ist für diese Verwendung insbesondere der Einsatz von Verbindungen nach Formel I bzw. II oder III, wobei X steht für eine kationische polare Gruppe oder eine polymerisierbare Gruppe.

Dabei werden die erfindungsgemäß einzusetzenden Verbindungen für die Anwendung üblicherweise in entsprechend ausgelegte Zubereitungen eingebracht. Entsprechende Mittel, die ebenfalls Gegenstand der vorliegenden Erfindung sind, enthalten mindestens eine oberflächenaktive Verbindung mit mindestens einer Endgruppe Y, wobei Y steht für CF₃O- oder F₅S- und einen für den jeweiligen Verwendungszweck geeigneten Träger sowie ggf. weitere spezifische Aktivstoffe und ggf. Hilfsstoffe.

Bei bevorzugten Mitteln handelt es sich dabei um Farb- und Lackzubereitungen, Feuerlöschmittel, Schmierstoffe, Wasch- und Reinigungsmittel, Enteiser oder Hydrophobiermittel zur Textilausrüstung oder Glasbehandlung. In einer bevorzugten Erfindungsvariante handelt es sich bei den Mitteln um Hydrophobiermittel zur Ausrüstung von Textilien und Teppichen.

Zur hydrophoben Ausrüstung von Textilien werden in der Regel Hydrophobierungsmittel auf Basis von Polysiloxanen, Fluorkohlenwasserstoffen oder Mischungen von Aluminium- oder Zirkonsalzen mit Paraffinen eingesetzt (vergleiche dazu "Handbuch der Textilhilfsmittel", A. Chwala, V. Anger, Verlag Chemie, New York 1977, Kapitel 3.24 "Phobiermittel", Seite 735 ff). Die hydrophobe Ausrüstung von Textilien, insbesondere bei Wetterschutzbekleidung, dient dazu, diese entweder wasserabweisend oder wasserundurchlässig zu machen. Das Hydrophobiermittel wird auf die Fasern der Textilien aufgebracht und ordnet sich dort so an, dass die hydrophoben Molekülteile senkrecht zur Faseroberfläche stehen. Auf diese Weise wird das Bestreben des Wassers, sich über die ganze Fläche auszubreiten, stark herabgesetzt. Das Wasser nimmt aufgrund der Kohäsionskräfte die Kugelform an und perlt von der Textiloberfläche ab.

Weitere Anwendungsgebiete für erfindungsgemäße Mittel sind Farb- und Lackzubereitungen, Feuerlöschmittel (Pulver und Schäume), Schmierstoffe, Wasch- und Reinigungsmittel und Enteiser.

Die Herstellung der erfindungsgemäß zu verwendenden Verbindungen kann dabei nach dem Fachmann an sich aus der Literatur bekannten Methoden erfolgen. Die aliphatische OCF₃-Gruppe kann beispielsweise aus Alkoholen über die Fluorodesulfurierung von Xanthogenaten erhalten werden (K. Kanie, Y. Tanaka, K. Suzuki, M. Kuroboshi, T. Hiyama, Bull. Chem. Soc. Jpn. 2000, 73, 471-484; P. Kirsch, Modem Fluoroorganic Chemistry: Synthesis, Reactivity, Applications, Wiley-VCH, Weinheim, 2004, S. 67 ff., S. 144 f.). Die entsprechende Offenbarung zu der genannten Methode in den zitierten Literaturstellen gehört damit ausdrücklich auch zum Offenbarungsgehalt der vorliegenden Anmeldung. Die Einführung der hydrophilen, anionischen, kationischen, reaktiven oder polymerisierbaren Endgruppe ist über den entsprechenden ω-OCF₃-Alkohol nach dem Fachmann bekannten Methoden möglich. Beispiele sind dem folgenden Schema zu entnehmen: 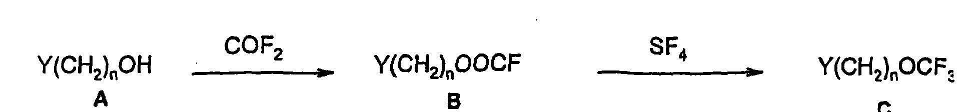 Y = Hal, OPg (Pg=Schutzgruppe) 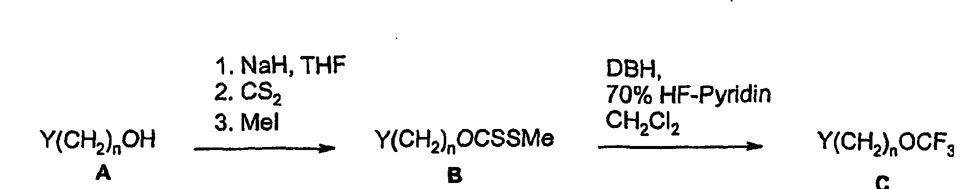 Y = Hal, OPg (Pg=Schutzgruppe)

### Derivatisierung für Y = OPg (z.B. OBn):

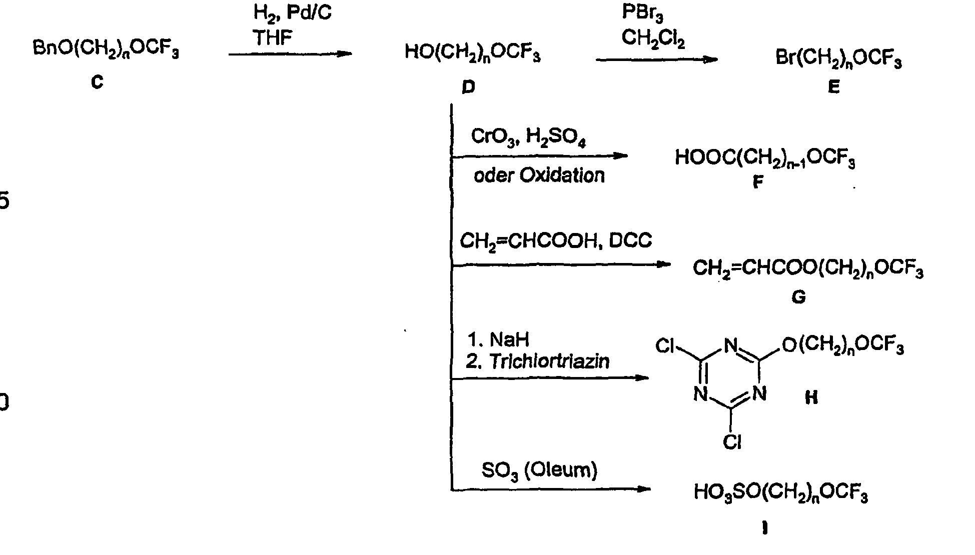

### Derivatisierung für Y = Hal (z.B. Br):

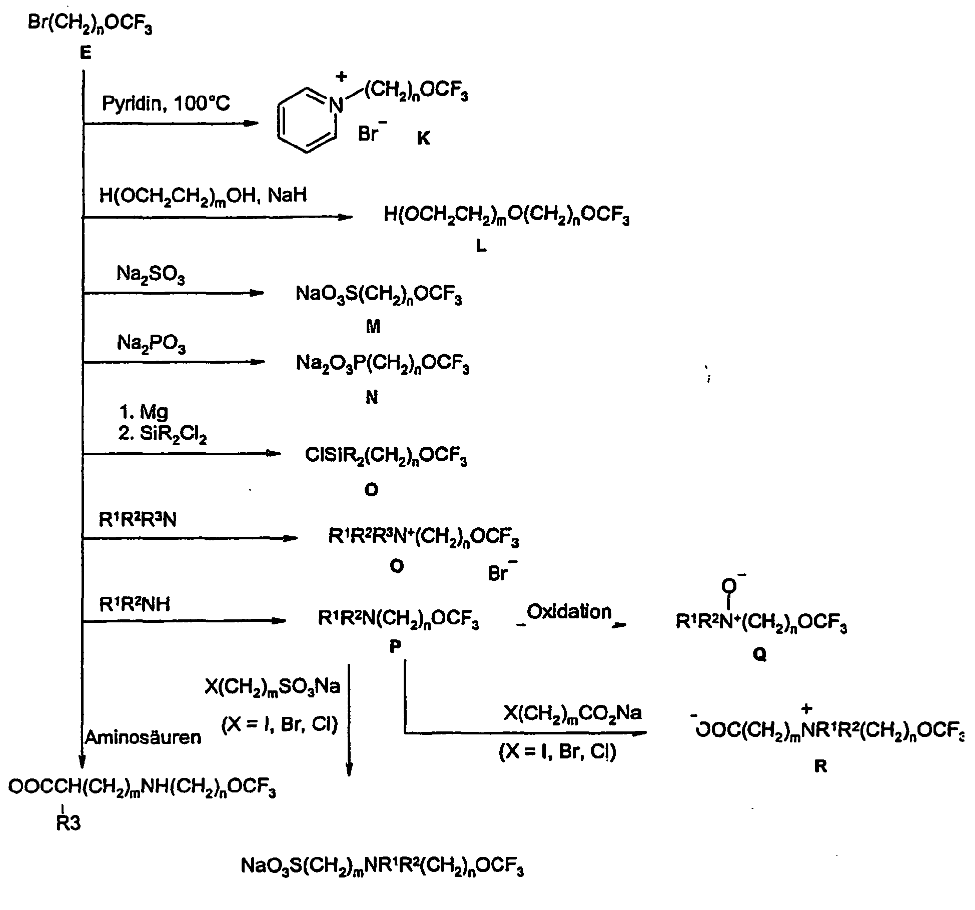

Die aliphatische SF₅-Gruppe kann z. B. an endständigen Doppelbindungen Ober die radikalische Addition von SF₅Cl oder SF₅Br eingefügt werden. Optionale anschließende Dehydrohalogenierung und Hydrierung erlauben die Variation der Endgruppen gemäß Formeln IIIa, IIIb bzw. IIIc. Die ersten beiden dieser Reaktionsschritte sind in der Literatur beschrieben (R. Winter, P. G. Nixon, G. L. Gard, D. H. Radford, N. R. Holcomb, D. W. Grainger, J. Fluorine Chem. 2001, 107, 23-30), katalytische Hydrierungen in Gegenwart einer SF₅-Funktion ebenfalls (P. Kirsch, M. Bremer, M. Heckmeier, K. Tarumi, Angew. Chem. 1999, 111, 2174-2178; Angew. Chem. Int. Ed. Engl. 1999, 38, 1989-1992). Die entsprechende Offenbarung zu der genannten Methode in den zitierten Literaturstellen gehört damit ausdrücklich auch zum Offenbarungsgehalt der vorliegenden Anmeldung. Die Einführung der hydrophilen, reaktiven oder polymerisierbaren Komponente ist über den entsprechenden ω-SF₅-Alkohol nach dem Fachmann bekannten Methoden möglich. Beispiele sind dem folgenden Schema zu entnehmen:

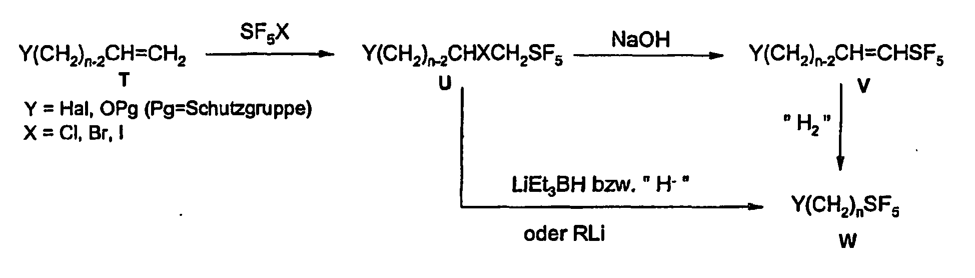

### Derivatisierung für Y = OPg (z.B. OBn):

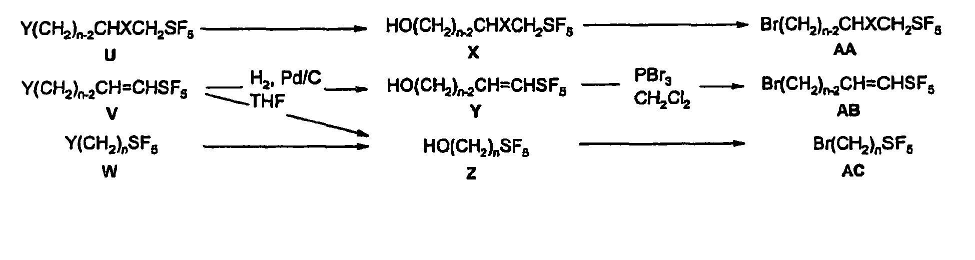

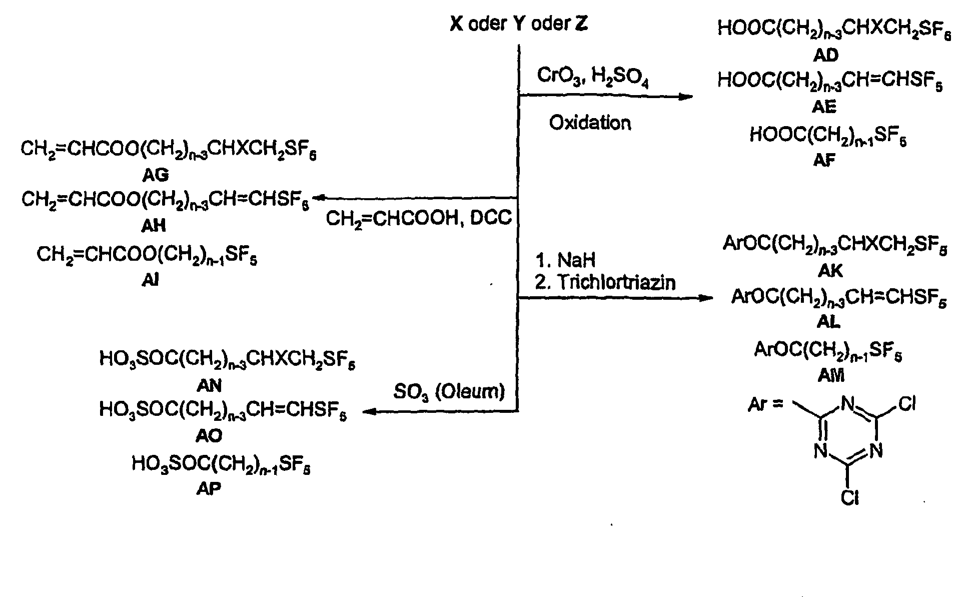

### Derivatisierung für Y = Hal (z.B. Br):

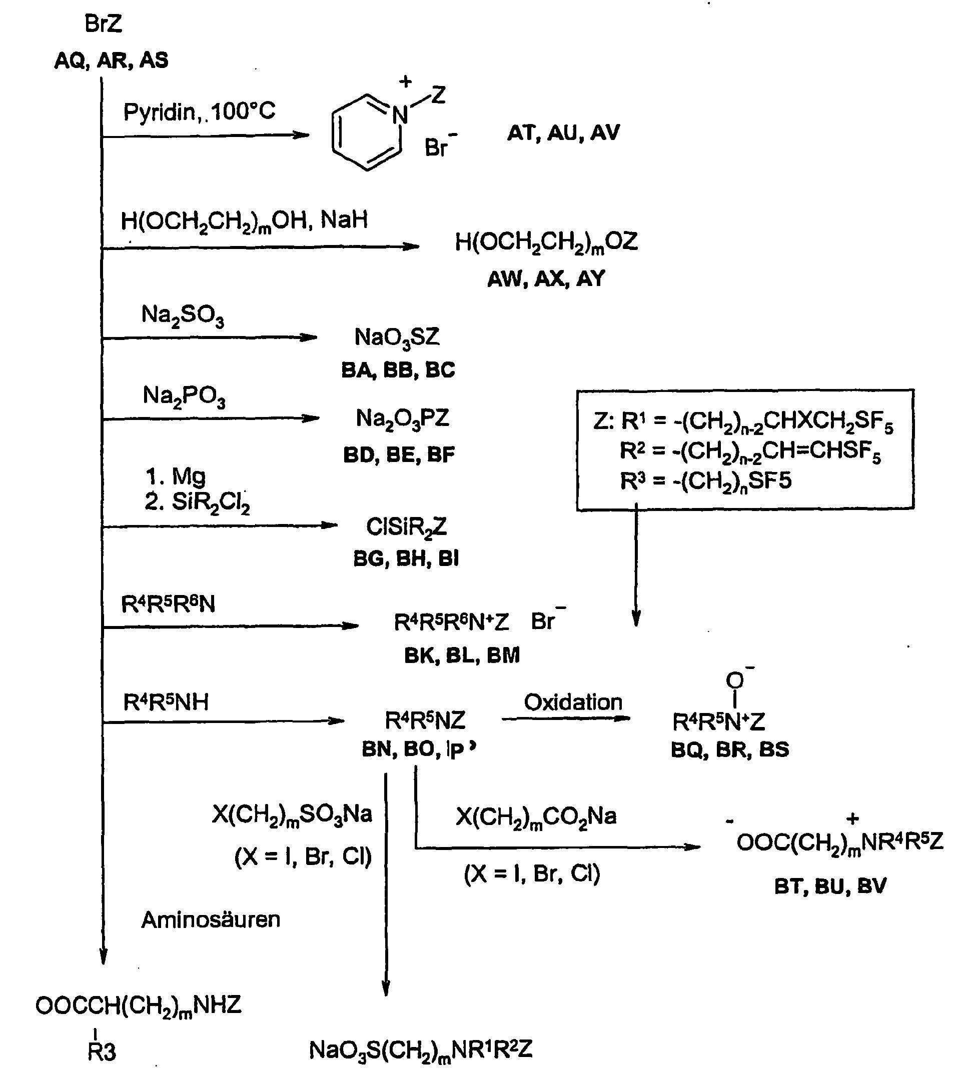

Die Auswahl geeigneter Lösungsmittel und Reaktionsbedingungen bereitet dem Fachmann dabei keinerlei Schwierigkeiten (Organikum: Organisch-Chemisches Grundpraktikum, 16. Aufl., VEB Deutscher Verlag der Wissenschaften, Berlin, 1986).

Weitere Gegenstände der vorliegenden Erfindung sind daher ein Verfahren zur Herstellung einer Verbindung nach Formel I, dadurch gekennzeichnet, dass zuerst eine Verbindung der Formel IV

Y-Spacer-OH IV

hergestellt wird und diese dann, soweit X in der Verbindung der Formel I verschieden von OH ist, durch Modifikation der OH-Gruppe in an sich bekannter Weise zur Verbindung der Formel I umgesetzt wird,
ein Verfahren zur Herstellung einer Verbindung nach Formel IIa,
dadurch gekennzeichnet, dass zuerst eine Verbindung der Formel V

F₃CO-(CH₂)ₙ-OH V

durch Umsetzung eines geschützten Diols zum geschützten Monotrifluormethoxy-alkohol und anschließendes Entschützen hergestellt wird und diese dann, soweit X in der Verbindung der Formel Ia verschieden von OH ist, durch Modifikation der OH-Gruppe in an sich bekannter Weise zur Verbindung der Formel IIa umgesetzt wird, und
ein Verfahren zur Herstellung einer Verbindung nach Formel IIIa, IIIb bzw. IIIc, dadurch gekennzeichnet, dass zuerst eine Verbindung der Forme IIIb, in der X steht für OH hergestellt wird und diese, wenn eine Verbindung der Formel IIIa bzw. IIIc hergestellt werden soll, durch Halogenwasserstoff-Eliminierung und, wenn eine Verbindung der Formel IIIa hergestellt werden soll, anschließende Hydrierung umgesetzt wird und anschließend, soweit X in der Verbindung nach Formel IIIa, IIIb bzw. IIIc verschieden von OH ist, das Produkt durch Modifikation der OH-Gruppe in an sich bekannter Weise zur Verbindung nach Formel IIIa, IIIb bzw. IIIc umgesetzt wird.

Die folgenden Beispiele erläutern die vorliegende Erfindung näher, ohne den Schutzbereich zu beschränken. Insbesondere sind die in den Beispielen beschriebenen Merkmale, Eigenschaften und Vorteile der den betreffenden Beispielen zugrunde liegenden Verbindungen auch auf andere nicht im Detail aufgeführte, aber unter den Schutzbereich fallende Stoffe und Verbindungen anwendbar, sofern an anderer stelle nicht gegenteiliges gesagt wird. Im übrigen ist die Erfindung im gesamten beanspruchten Bereich ausführbar und nicht auf die hier genannten Beispiele beschränkt.

### Beispiele

Verzeichnis der verwendeten Abkürzungen:
Bn: Benzyl
DBH: 1,3-Dibrom-5,5-dimethylhydanthoin
DMAP: 4-(Dimethylamino)pyridin
Me: Methyl
THF: Tetrahydrofuran

### Beispiel 1: ω-Trifluormethoxyalkanole

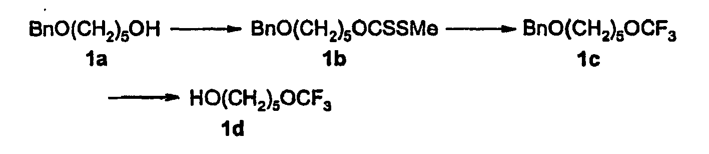

**1b**: Zu einer Suspension von 95 mmol NaH in 400 ml THF werden bei 0°C 80 mmol **1a** in 200 ml THF getropft. Man erhitzt für 3 h auf 40°C, kühlt wieder auf 0°C und tropft 160 mmol CS₂ zu. Nach 1 h Nachrühren bei RT werden 95 mmol Mel bei 0°C zugetropft. Man rührt 18 h bei RT, arbeitet wie üblich wässrig auf und chromatographiert über Kieselgel.
**1c**: Eine Suspension von 30 mmol DBH (1,3-Dibrom-5,5-dimethylhydanthoin) in 30 ml CH₂Cl₂ in einem Teflongefäß wird bei -78°C langsam mit 20 ml 70% HF-Pyridin versetzt. Zu dieser Mischung wird bei -78°C eine Lösung von 10 mmol **1b** in 30 ml CH₂Cl₂ getropft. Man rührt 30 min bei -78°C, lässt dann auf 0°C kommen und hydrolysiert mit NaHSO₃-Lösung. Man arbeitet wässrig auf und chromatographiert über Kieselgel.
**1d**: 20 mmol **1c** in 400 ml THF werden in Gegenwart von 2 g 5% Pd-C bei RT und 4 bar Druck bis zum Ende der Wasserstoffaufnahme hydriert. Man filtriert Ober Celite vom Katalysator ab, und reinigt das Produkt durch fraktionierte Destillation.

Durch Variation der Alkyl-Kettenlänge der Edukte können entsprechende Derivate hergestellt werden.

### Beispiel 2: ω-Pentafluorsulfuranylalkanole

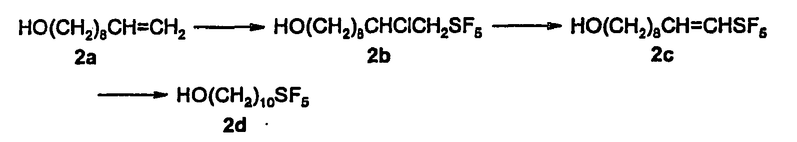

**2a** und **2b** werden entsprechend der Vorschrift in R. Winter, P. G. Nixon, G. L. Gard, D. H. Radford, N. R. Holcomb, D. W. Grainger, J. Fluorine Chem. 2001, 107, 23-30 hergestellt.
**2c**: 20 mmol **2b** in 400 ml THF werden in Gegenwart von 2 g 5% Pd-C bei RT und Normaldruck bis zum Ende der Wasserstoffaufnahme hydriert. Man filtriert über Celite vom Katalysator ab, und reinigt das Produkt durch fraktionierte Destillation.

Durch Variation der Alkyl-Kettenlänge der Edukte können entsprechende Derivate hergestellt werden.

### Beispiel 3: Oberflächenaktive Substanzen mit terminalen Trifluormethoxy- und Pentafluorsulfuranylgruppen

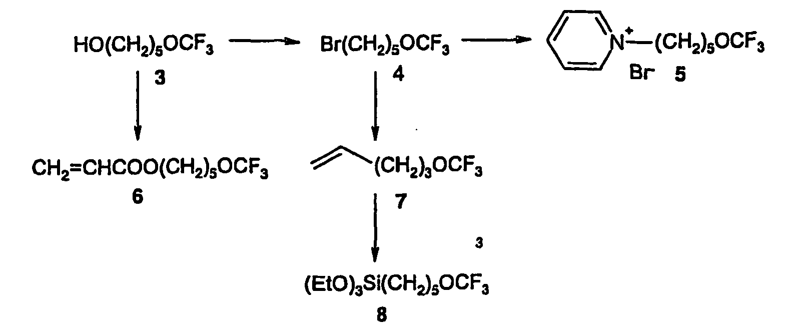

**4:** Eine Lösung von 100 mmol **3** in 200 ml Toluol wird unter Eiskühlung mit 120 mmol PBr₃ versetzt und für 3h zum Rückfluss erhitzt. Man lässt abkühlen und arbeitet wie üblich wässrig auf. Das Rohprodukt wird destillativ gereinigt.
**5:** Eine Mischung von 10 mmol **4** und 30 ml Pyridin wird für 2 h zum Sieden erhitzt. Man engt im Vakuum ein und fällt den Rückstand mit Diethylether aus.
**6:** Eine Lösung von 100 mmol **3,** 100 mmol Acrylsäure, 105 mmol Dicyclohexylcarbodiimid und 5 mmol DMAP in 300 ml THF wird 18 h bei RT gerührt. Man gießt in Wasser und arbeitet wie üblich wässrig auf. Das Rohprodukt wird durch Chromatographie auf Kieselgel (Pentan) gereinigt.
**7:** Eine Mischung von 100 mmol **4** und 150 mmol Dicyclohexylamin wird für 18 h auf 180°C erhitzt. Das Produkt wird abdestilliert, gegen Ende bei verringertem Druck und erhöhter Temperatur. Das Rohprodukt wird durch fraktionierte Destillation gereinigt.
**8:** Eine Lösung von 10 mmol **7** und 20 mmol Triethoxysilan in 80 ml CH₂Cl₂ wird mit 16 mg H₂PtCl₆·6H₂O in 1 ml *iso*-Propanol versetzt und 4 d bei RT gerührt. Nach beendeter Umsetzung wird das Produkt destillativ gereinigt.

Durch Variation der Alkyl-Kettenlänge der Edukte können entsprechende Derivate hergestellt werden.

### Beispiel 4:

Durch Umsetzungen analog zu Beispiel 3 werden aus 5-Pentafluorsulfuranyl-pentanol die entsprechenden Pentafluorsulfuranyl-Derivate erhalten. Durch Variation der Alkyl-Kettenlänge der Edukte können entsprechende Derivate hergestellt werden.

### Beispiel 5: ω-Trifluormethoxyalkansulfonat

### Beispiel 5a: Synthese von Dithiocarbonsäure (10-bromo-decyl)-ester methyl-ester:

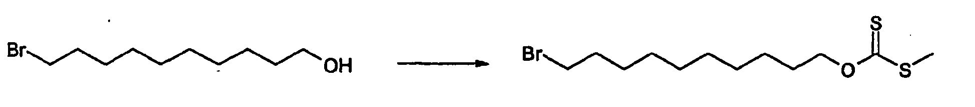

In einer mit Stickstoff begaste 1I Vierhals-Glasapparatur werden 200 ml Tetrahydrofuran (THF) + 10,15 g NaH (253mmol, 1.2 eq) vorgelegt und auf -25°C abgekühlt. 50 g 6-Bromo-1-decanol (211 mmol, 1 eq) werden, mit 100 ml THF gemischt, unter Kühlung zugetropft. Es wird 120 min. bei RT nachgerührt und anschließend wiederum auf -25°C gekühlt. Schwefelkohlenstoff (32.1 g; 421.6 mmol; 2 eq) wird zugetropft und anschließend 2,5 h bei 0°C nachgerührt. Bei -20°C wird Methyliodid (35.9g; 253 mmol; 1.2 eq) unter Kühlung zugetropft. Die Reaktionsmischung wird langsam auf RT erwärmt und 24 h nachgerührt. Nach Quenchen mit 10%iger NH₄Cl-Lösung ( 200 ml) werden die Phasen getrennt und die organische Phase gewaschen und zur Trockne eingeengt.

### Beispiel 5b: Synthese von 1-Bromo-10-trifluoromethoxy-decan:

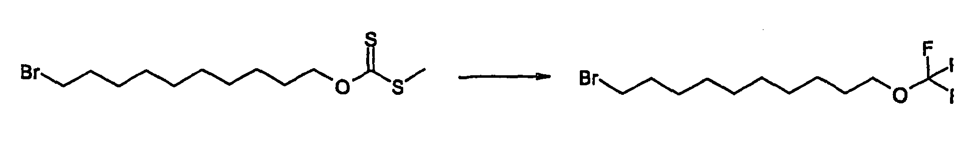

Zu einer Suspension aus 1,3-Dibrom-5,5-dimethylhydantoin (102g; 119 mmol; 3eq) in 420 ml Dichlormethan werden bei -76°C 200 ml (7250 mmol, 61 eq) HF/Pyridin (65-70%ig) zugetropft und nachgerührt. Danach wird das Xanthogenat aus Beispiel 5a (37g; 118.9 mmol; 1 eq) in 50 ml Dichlormethan zugetropft. Die Reaktionsmischung wird 12 h bei RT gerührt. Mit wässriger KOH-Lösung wird pH = 10 eingestellt. Die Reaktionsmischung wurde mit Wasser und Methyl-t-butyl-ether verdünnt und anschließend abfiltriert. Die organische Phase wird getrocknet und säulenchromatographisch mit Heptan gereinigt.

### Beispiel 5c: Synthese von 10- Trifluoromethoxy-decan-1-sulfonsäure:

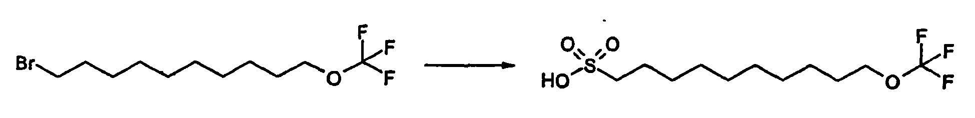

In einem 250ml Einhalskolben werden 6.8g Bromid aus Beispiel 5b (22.35 mmol) und 3.7g (29 mmol; 1.3 eq) Natriumsulfit in 40 ml VE-Wasser und 40 ml Ethanol gelöst und 20 h auf 100°C erhitzt. Die abgekühlte Reaktionsmischung wird mit Methyl-t-butyl-ether /Heptan (1:1) extrahiert. Die wässrige Phase angesäuert (pH=0) und mit Methyl-t-butyl-ether extrahiert. Die vereinten organischen Phasen wurden über Natriumsulfat getrocknet und eingeengt.

### Beispiel 5d: Synthese von Natrium -10-trifluoromethoxy-decan-1-sulfonat:

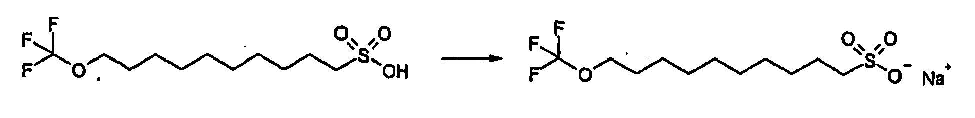

1.15g (28.8 mmol; 1.3 eq) NaOH werden in 60 ml Ethanol gelöst und bei RT zur Sulfonsäure aus Beispiel 5c (6.8g; 22.2 mmol; 1 eq) gegeben. Nach 1 h unter Rückfluss wird ein farbloser Feststoff gewonnen.

Analog Beispiel 5 können allgemein Sulfonate mit verschiedenen Alkylen-Kettenlängen erhalten werden.

### Beispiel 6: ω-Pentafluorosulfuranyl-(ω-1)-Chloro-heptan-1-sulfonat

### Beispiel 6a: Synthese von 1-Bromo-6-chloro-7-(pentafluorosulfuranyl)-heptan:

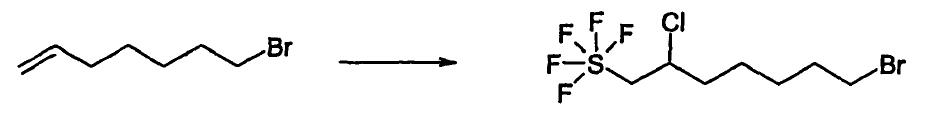

In 170 mL Dichlormethan werden 10g (56.5mmol; 1 eq) 7-Bromhepten gelöst und auf -40°C eingekühlt. SF5Cl wird in einer Kühlfalle einkondensiert und als Gas in die Apparatur eingeleitet. Zur Aktivierung werden 2 mL 1-M-Et₃B Lösung zugegeben. Bei der Gaseinleitung ist der Ansatz gelb, nach Et₃B Zugabe wird der Ansatz farblos. Die Zugabe wird so oft wiederholt bis sich der Ansatz nicht mehr entfärbt. Anschließend wird das Reaktionsgemisch zwei Stunden nachgerührt. Das Reaktionsgemisch wird hydrolisiert und pH = 10 eingestellt. Die organische Phase wird gewaschen und getrocknet.

### Beispiel 6b: Synthese von 6-Chloro-7-(pentafluorosulfuranyl)-heptan-1-sulfonsäure:

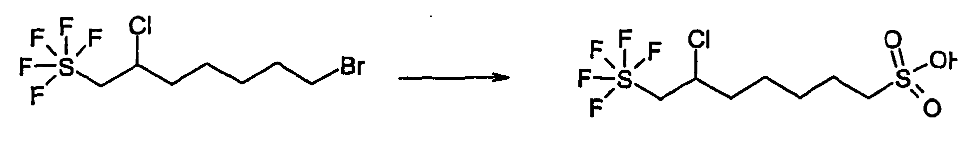

In einem 250ml Einhalskolben werden 10 g (29.4mmol; 1eq) des Produktes aus Beispiel 6a und 3.72g (38,3mmol; 1.3eq) Natriumsulfit in 50ml VE-Wasser und 50ml Ethanol gelöst und 15 h auf 100°C erhitzt. Das Reaktionsgemisch wird nach Abkühlen mit einem 1:1-Gemisch aus Methyl-t-butyl-ether und Heptan extrahiert. Die wässrige Phase wird angesäuert und mit Methyl-t-butyl-ether extrahiert. Die vereinigten organischen Phasen werden gewaschen und zur Trockne eingeengt.

### Beispiel 6c: Synthese von Natrium 6-Chioro-7-(pentafluorosulfuranyl)-heptan-1-sulfonat:

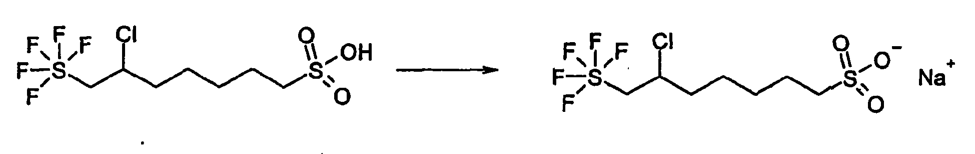

10g (29.35mmol; 1 eq) Sulfonsäure aus Beispiel 6b werden In 130 ml Ethanol suspendiert und 1.4g (35.22mmol; 1.2eq) Natriumhydroxid zugegeben. Die Reaktionsmischung wird 1 h unter Rückfluss erhitzt. Der Feststoff wird bei RT abfiltriert.

### Beispiel 7: Synthese von Natrium 7-(Pentafluorosulfuranyl)-hept-6-en-1-sulfonat:

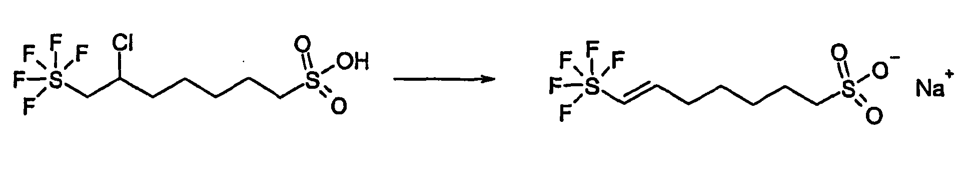

In einem 500 ml Kolben wurden 10g (29.35mmol; 1eq) Sulfonsäure aus Beispiel 6b In 400 ml Tetrahydrofuran suspendiert und anschließend 11.74g (293.5mmol; 10eq) Natriumhydroxid zugegeben. Die Reaktionsmischung wird 10 h unter Rückfluss erhitzt, nach Abkühlen angesäuert und die Sulfonsäure durch mehrmaliges Extrahieren mit Methyl-t-butyl-ether von der wässrigen Phase abgetrennt. Anschließend wird die organische Phase gewaschen und zur Trockne eingeengt. Die rohe Sulfonsäure wird in 100ml Ethanol suspendiert und mit 1,4g (35.22mmol;1.2 eq) Natriumhydroxid versetzt und 1 h auf 97°C erhitzt. Nach Abkühlen der Suspension fallen Kristalle aus, die abfiltriert und getrocknet werden.

### Beispiel 8: Bestimmung der biochemischen Abbaubarkeit

Die biochemische Abbaubarkeit der Verbindungen wird nach dem Zahn-Wellens-Test entsprechend der Publikation der Europäischen Kommission: Einstufung, Verpackung und Kennzeichnung gefährlicher Stoffe in der Europäischen Union, Teil II - Testmethoden, Anhang V - Methoden zur Bestimmung der physikalisch-chemischen Eigenschaften, der Toxizität und der Ökotoxizität, Teil B, Biochemische Abbaubarkeit- Zahn-Wellens-Test(C.9.), Januar 1997, Seite 353-357 bestimmt.

| | |
|---|---|
| Ansatzvolumen: | 1,5 l |
| Belebtschlammkonzentration: | 1 gTS/l |
| Herkunft des Schlammes: | Kläranlage der Merck KGaA; |
| | Darmstadt (nicht adaptiert) |
| Einsatzmenge der Testsubstanzen: | ca. 100 bis 200 mg/l als DOC |
| Belüftung: | mit gereinigter Luft |
| Aufarbeitung der Proben: | Filtration (mittelhartes Filter) |
| Bestimmung des DOC: | Nach der Differenzmethode mit einem Gerät der Fa. Dimatec |

Weitere Details zur Methode können der o.g. Publikation bzw. auch der OECD Guideline for the testing of chemicals, section 3, degradation and accumulation, method 302 B, page 1-8, adopted: 17.07.92 entnommen werden, deren diesbezüglicher Inhalt ausdrücklich zum Offenbarungsgehalt der vorliegenden Anmeldung gehört.

Darüberhinaus wird neben dem Abbau der Verbindung an sich im Test auch der Abbau der Fluor-haltigen Gruppen über eine Fluorid-Bestimmung beobachtet:

| | |
|---|---|
| Methode: | lonenchromatographie |
| Gerät: | Dionex 120 |
| Detektortyp: | Leitfähigkeitsdetektor |
| Säule: | AS9HC |
| Eluent: | Natriumcarbonat-Lösung, 9 mmol/l |
| Flussrate: | 1 ml/min |
| Literatur: | EN ISO 10304-2 |

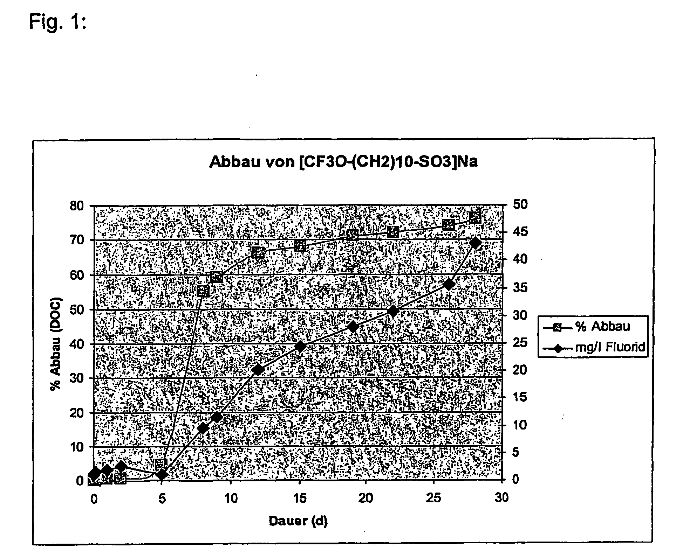

Untersucht wird das Natrium -10-trifluoromethoxy-decan-1-sulfonat aus Beispiel 5. Die Messwerte sind in der folgenden Tabelle wiedergegeben und in Figur 1 graphisch dargestellt.

| **Dauer der Messung (d)** | mg/l freies Fluorid | **Abbau (DOC) in %** |
|---|---|---|
| 0 | 1,1 | 0 |
| 0,125 | 1,6 | 0 |
| 1 | 2,0 | 0,5 |
| 2 | 2,6 | 0,7 |
| 5 | 1,1 | 4,5 |
| 8 | 9,6 | 55 |
| 9 | 11,5 | 59 |
| 12 | 20,3 | 66 |
| 15 | 24,5 | 68 |
| 19 | 28,1 | 71 |
| 22 | 30,9 | 72 |
| 26 | 35,7 | 74 |
| 28 | 43,1 | 76 |

Es zeigt sich, dass die Verbindung, unter Beteiligung der fluorierten CF₃O-Gruppe, unter den Versuchsbedingungen biologisch abgebaut wird.

### Beispiel 9: Bestimmung der Oberflächenspannung:

| | |
|---|---|
| Gerät: | Tensiometer der Firma Krüss (Modell K12) |
| Temperatur der Messlösungen: | 20°C |
| Eingesetztes Messmodul: | Ring |
| Konzentration der Messlösungen: | ca. 0,5 bis 3,0 g/l in entionisiertem |
| Wasser | |

Weitere Details zur Methode können der Publikation Europäische Kommission: Einstufung, Verpackung und Kennzeichnung gefährlicher Stoffe in der Europäischen Union, Teil II - Testmethoden, Anhang V - Methoden zur Bestimmung der physikalisch-chemischen Eigenschaften, der Toxizität und der Ökotoxizität, Teil A, Oberflächenspannung (A.5), Januar 1997, Seite 51-57 bzw. auch der OECD Guideline for the testing of chemicals , section 1, physical-chemical properties, method 115, page 1-7, adopted: 27.07.95 entnommen werden, deren diesbezüglicher Inhalt ausdrücklich zum Offenbarungsgehalt der vorliegenden Anmeldung gehört.

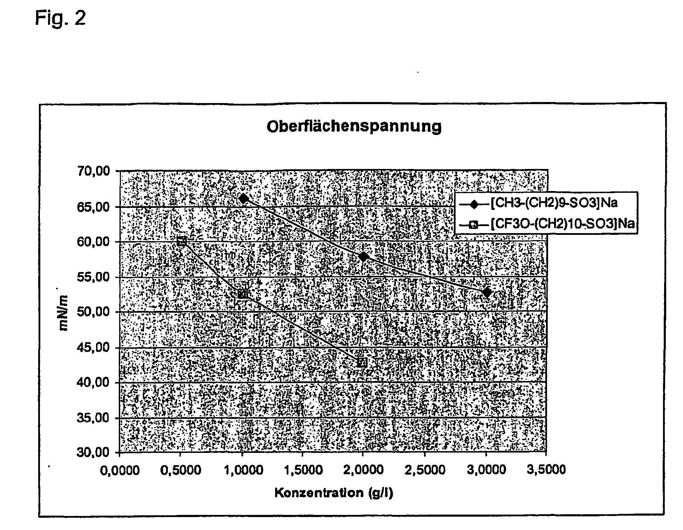

Untersucht wird das Natrium -10-trifluoromethoxy-decan-1-sulfonat aus Beispiel 5 im Vergleich zu dem klassischen Kohlenwasserstofftensid Natrium-decansulfonat. Die Messwerte sind in der folgenden Tabelle wiedergegeben und in Figur 2 graphisch dargestellt.

| Probe | Konzentration der Lösung | Konzentration der Lösung | Oberflächenspannung |
|---|---|---|---|
| | g/l | mol/l | mN/m |
| Beispiel 5 | 0,5015 | 1,527E-03 | 59,95 |
| | 1,0000 | 3,046E-03 | 52,55 |
| | 2,0005 | 6,093E-03 | 42,61 |
| Decansul- | 1,0020 | 4,101E-03 | 66,13 |
| fonsäure | 2,0015 | 8,192E-03 | 57,70 |
| Na-salz | 3,0010 | 1,228E-02 | 52,61 |

Es zeigt sich, dass das erfindungsgemäße Tensid im Vergleich zum Kohlenwasserstofftensid, die gleiche Oberflächenspannung bei deutlich niedrigerer Konzentration erzeugt. Zudem lässt die Kurvenextrapolation vermuten, dass auch der Endwert bei dem erfindungsgemäßen Tensid deutlich niedriger liegen dürfte als bei dem Kohlenwasserstofftensid.

### Verzeichnis der Figuren

Figur 1 gibt die biochemische Abbaubarkeit von Natrium -10-trifluoromethoxy-decan-1-sulfonat im Zahn-Wellems-Test (DOC-Werte) und die Fluorid-Freisetzung, während des Tests, gemäß Beispiel 8 an.
Figur 2 zeigt die Veränderung der Oberflächenspannung von Wasser in Abhängigkeit zur Tensidkonzentration für Natrium -10-trifluoromethoxydecan-1-sulfonat und Natrium-decansulfonat gemäß Beispiel 9 an.

## Patentansprüche

1. Verwendung von Verbindungen enthaltend mindestens eine hydrophobe Endgruppen Y, wobei Y steht für CF₃O- oder F₅S-, die Endgruppe Y an eine gesättigte oder ungesättigte, verzweigte oder unverzweigte Kohlenwasserstoff-Einheit gebunden ist und Verbindungen verwendet werden, die der Formel Ia, Ib oder Ic
Y-(CH₂)ₙ-X Ia
Y-CH₂-CH(Hal)-(CH2)₍ₙ₋₁₎-X Ib
Y-CH=CH-(CH₂)₍ₙ₋₁₎-X Ic
worin Y steht für CF₃O- oder F₅S-,
n steht für eine ganze Zahl aus dem Bereich 4 bis 28 und
X steht für eine kationische, nichtionische, ampothere oder anionische polare Gruppe oder eine polymerisierbare Gruppe, (Hal) steht für F, Cl, Br oder I,
sowie entsprechender Salze der Verbindungen nach Formel Ia, Ib bzw. Ic
entsprechen, als oberflächenaktive Mittel.

2. Verwendung nach Anspruch 1,
**dadurch gekennzeichnet, dass** die oberflächenaktive Verbindung als Tensid verwendet wird.

3. Verwendung nach Anspruch 1,
**dadurch gekennzeichnet, dass** die oberflächenaktive Verbindung als Hydrophobiermittel verwendet wird.

4. Verwendung nach Anspruch 1,
**dadurch gekennzeichnet, dass** die oberflächenaktive Verbindung als Grenzflächenvermittler verwendet wird.

5. Verwendung nach Anspruch 1,
**dadurch gekennzeichnet, dass** die oberflächenaktive Verbindung als Viskositätsminderer, insbesondere in Farben, Lacken oder Zubereitungen zur Oberflächenbeschichtung verwendet wird.

6. Verwendung nach Anspruch 1,
**dadurch gekennzeichnet, dass** die oberflächenaktive Verbindung als Schaumstabilisator, insbesondere in Feuerlöschschäumen verwendet wird.

7. Verwendung nach Anspruch 1,
**dadurch gekennzeichnet, dass** die oberflächenaktive Verbindung als Emulgator, insbesondere für die Herstellung von Fluorpolymeren verwendet wird.

8. Verwendung nach Anspruch 1,
**dadurch gekennzeichnet, dass** die oberflächenaktive Verbindung als antimikrobieller Wirkstoff, insbesondere als Reagenz für die antimikrobielle Oberflächenmodifikation verwendet wird.

9. Verwendung nach mindestens einem der vorstehenden Ansprüche,
**dadurch gekennzeichnet, dass** X steht für eine anionische polare Gruppe ausgewählt aus -COOM, -SO₃M, -OSO₃M, -PO₃M₂, -OPO₃M₂, -(OCH₂CH₂)ₘ-O-(CH₂)ₒ-COOM, -(OCH₂CH₂)ₘ-O-(CH₂)ₒ-SO₃M, -(OCH₂CH₂)ₘ-O-(CH₂)ₒ-OSO₃M, -(OCH₂CH₂)ₘ-O-(CH₂)ₒ-PO₃M₂, -(OCH₂CH₂)ₘ-O-(CH₂)ₒ-OPO₃M₂,
wobei M steht für H oder ein Alkalimetall-lon, vorzugsweise Li⁺, Na⁺ oder K⁺, oder NH₄⁺,
m steht für eine ganze Zahl aus dem Bereich von 1 bis 1000 und
o steht für eine ganze Zahl ausgewählt aus 1, 2, 3 oder 4.

10. Verwendung nach mindestens einem der Ansprüche 1 bis 9,
**dadurch gekennzeichnet, dass** X steht für eine kationische polare Gruppe ausgewählt aus -NR¹R²R^{3 +} Z⁻, -PR¹R²R³⁺ Z⁻, 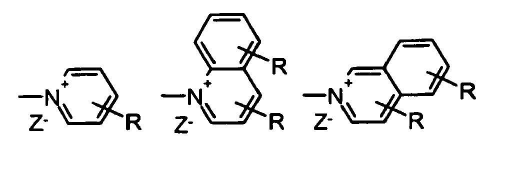 wobei R steht für H oder C₁₋₄-Alkyl in beliebiger Position,
Z⁻ steht für Cl⁻, Br⁻, I⁻, CH₃SO₃⁻, CF₃SO₃⁻, CH₃PhSO₃⁻, PhSO₃⁻
R¹, R² und R³ jeweils unabhängig voneinander stehen für H, C₁₋₃₀-Alkyl, Ar oder -CH₂Ar und
Ar steht für einen unsubstituierten oder ein- oder mehrfach substituierten aromatischen Ring oder kondensierte Ringsysteme mit 6 bis 18 C-Atomen, worin auch ein oder zwei CH-Gruppen durch N ersetzt sein können.

11. Verwendung nach mindestens einem der Ansprüche 1 bis 9,
**dadurch gekennzeichnet, dass** X steht für eine nicht-ionische polare Gruppe ausgewählt aus -Cl, -Br, -I, -(OCH₂CH₂)ₘ-OH, -O-(Glycosid)ₒ, -(OCH₂CH₂)ₘ-OCH₂-CHOH-CH₂-OH, -(OCH₂CH₂)ₘ-OCH₂Ar(-NCO)ₚ, -(OCH₂CH₂)ₘ-OAr(-NCO)ₚ , -SiR¹R²Z, -SiR¹Z₂, -SiZ₃, -R2-COZ, -(OCH₂CH₂)ₘ-SO₂CH=CH₂, -SO₂Z; 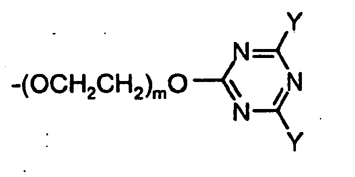
m steht für eine ganze Zahl aus dem Bereich von 0 bis 1000,
n steht für 0 oder 1 und
o steht für eine ganze Zahl aus dem Bereich von 1 bis 10,
p steht für 1 oder 2,
R¹ und R² jeweils unabhängig voneinander stehen für C₁₋₃₀-Alkyl, Ar oder -CH₂Ar und
Ar steht für einen unsubstituierten oder ein- oder mehrfach substituierten aromatischen Ring oder kondensierte Ringsysteme mit 6 bis 18 C-Atomen, worin auch ein oder zwei CH-Gruppen durch N ersetzt sein können, und
Glycosid steht für ein verethertes Kohlenhydrat, vorzugsweise für ein mono- di-, tri- oder oligo-Glucosid,
alle Z jeweils unabhängig voneinander stehen für -H, -Cl, -F, -NR¹R², -OR¹, -N-Imidazolyl und
Y steht für Cl oder F.

12. Verwendung nach mindestens einem der Ansprüche 1 bis 9,
**dadurch gekennzeichnet, dass** X steht für eine polymerisierbare Gruppe ausgewählt aus -(OCH₂CH₂)ₘOCOCR=CH₂, -(OCH₂CH₂)ₘ-OCR=CH₂, 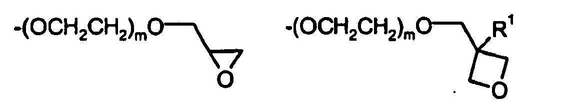 wobei m steht für eine ganze Zahl aus dem Bereich von 0 bis 1000 und R bzw. R¹ steht für H oder C₁₋₄-Alkyl.

13. Verwendung nach mindestens einem der Ansprüche 1 bis 9,
**dadurch gekennzeichnet, dass** X steht für eine amphotere Gruppe ausgewählt aus den funktionellen Gruppen der Acetyldiamine, der N-Alkylaminosäuren, der Betaine, der Aminoxide bzw. entsprechender Derivate davon.

14. Verbindung nach Formel IIa, IIb bzw. IIc
CF₃O-(CH₂)ₙ-X IIa
CF₃O-CH₂-CH(Hal)-(CH₂)₍ₙ₋₁₎-X IIb
CF₃O-CH=CH-(CH₂)₍ₙ₋₁₎-X IIc
worin
n steht für eine ganze Zahl aus dem Bereich 4 bis 28
X steht für eine kationische, nichtionische, amphotere oder anionische polare Gruppe oder eine polymerisierbare Gruppe,
(Hal) steht für F, Cl, Br oder I,
sowie entsprechende Salze der Verbindungen nach Formel IIa, IIb bzw. IIc, wobei die Verbindung CF₃-O-CH₂-COOH ausgenommen ist.

15. Verbindung nach Formel IIIa, IIIb oder IIIc
F₅S-(CH₂)ₙ-X IIIa
F₅S-CH₂-CH(Hal)-(CH₂)₍ₙ₋₁₎-X IIIb
F₅S-CH=CH-(CH₂)₍ₙ₋₁₎-X IIIc
worin
n steht für eine ganze Zahl aus dem Bereich 4 bis 28 und
X steht für eine kationische, nichtionische, ampothere oder anionische polare Gruppe oder eine polymerisierbare Gruppe,
(Hal) steht für F, Cl, Br oder I,
sowie entsprechende Salze der Verbindungen nach Formel IIIa, IIb bzw. IIIc, wobei die Verbindungen F₅S-CH₂-CHCl-(CH₂)₈-X¹ wobei X¹ steht für OH, OC(=O)CH₃, Br, C(=O)OC₂H₅, C(=O)CH₃, ausgenommen sind.

16. Verbindung nach mindestens einem der Ansprüche 14 oder 15,
**dadurch gekennzeichnet, dass** X steht für eine anionische polare Gruppe ausgewählt aus -COOM, -SO₃M, -OSO₃M, -PO₃M₂, -OPO₃M₂, -(OCH₂CH₂)ₘ-O-(CH₂)ₒ-COOM, -(OCH₂CH₂)ₘ-O-(CH₂)ₒ-SO₃M, -(OCH₂CH₂)ₘ-O-(CH₂)ₒ-OSO₃M, -(OCH₂CH₂)ₘ-O-(CH₂)ₒ-PO₃M₂, -(OCH₂CH₂)ₘ-O-(CH₂)ₒ-OPO₃M₂, wobei M steht für H oder ein Alkalimetall-lon, vorzugsweise Li⁺, Na⁺ oder K⁺, oder NH₄⁺,
m steht für eine ganze Zahl aus dem Bereich von 1 bis 1000 und
o steht für eine ganze Zahl ausgewählt aus 1, 2, 3 oder 4.

17. Verbindung nach mindestens einem der Ansprüche 14 oder 15,
**dadurch gekennzeichnet, dass** X steht für eine kationische polare Gruppe ausgewählt aus -NR¹R²R³⁺ Z⁻, -PR¹R²R³⁺ Z⁻, 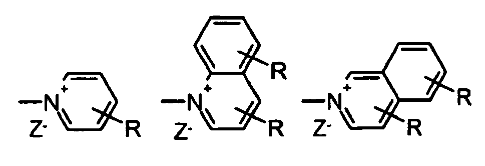 wobei R steht für H oder C₁₋₄-Alkyl in beliebiger Position,
Z⁻ steht für Cl⁻, Br⁻, I⁻, CH₃SO₃⁻, CF₃SO₃⁻, CH₃PhSO₃⁻, PhSO₃⁻
R¹, R² und R³ jeweils unabhängig voneinander stehen für H, C₁₋₃₀-Alkyl, Ar oder -CH₂Ar und
Ar steht für einen unsubstituierten oder ein- oder mehrfach substituierten aromatischen Ring oder kondensierte Ringsysteme mit 6 bis 18 C-Atomen, worin auch ein oder zwei CH-Gruppen durch N ersetzt sein können.

18. Verbindung nach mindestens einem der Ansprüche 14 oder 15,
**dadurch gekennzeichnet, dass** X steht für eine nicht-ionische polare Gruppe ausgewählt aus -Cl, -Br, -I, -(OCH₂CH₂)ₘ-OH, -O-(Glycosid)ₒ, -(OCH₂CH₂)ₘ-OCH₂-CHOH-CH₂-OH, -(OCH₂CH₂)ₘ-OCH₂Ar(-NCO)ₚ, -(OCH₂CH₂)ₘ-OAr(-NCO)ₚ, -SiR¹R²Z, -SiR¹Z₂, -SiZ₃, -COZ, -(OCH₂CH₂)ₘ-SO₂CH=CH₂, -SO₂Z, 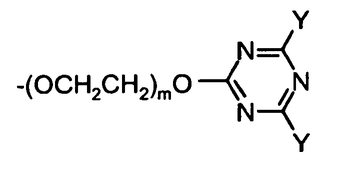
m steht für eine ganze Zahl aus dem Bereich von 0 bis 1000,
n steht für 0 oder 1 und
o steht für eine ganze Zahl aus dem Bereich von 1 bis 10,
p steht für 1 oder 2,
R¹ und R² jeweils unabhängig voneinander stehen für C₁₋₃₀-Alkyl, Ar oder -CH₂Ar und
Ar steht für einen unsubstituierten oder ein- oder mehrfach substituierten aromatischen Ring oder kondensierte Ringsysteme mit 6 bis 18 C-Atomen, worin auch ein oder zwei CH-Gruppen durch N ersetzt sein können und
Glycosid steht für ein verethertes Kohlenhydrat, vorzugsweise für ein mono- di-, tri- oder oligo-Glucosid,
alle Z jeweils unabhängig voneinander stehen für -H, -Cl, -F, -NR¹R², -OR¹, -N-Imidazolyl und
Y steht für Cl oder F.

19. Verbindung nach mindestens einem der Ansprüche 14 oder 15,
**dadurch gekennzeichnet, dass** X steht für eine polymerisierbare Gruppe ausgewählt aus -(OCH₂CH₂)ₘOCOCR=CH₂, -(OCH₂CH₂)ₘ-OCR=CH₂, 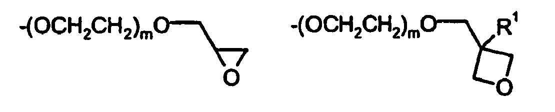 wobei m steht für eine ganze Zahl aus dem Bereich von 0 bis 1000 und R bzw. R¹ steht für H oder C₁₋₄-Alkyl.

20. Verbindung nach mindestens einem der Ansprüche 14 oder 15,
**dadurch gekennzeichnet, dass** X steht für eine amphotere Gruppe ausgewählt aus den funktionellen Gruppen der Acetyldiamine, der N.-Alkylaminosäuren, der Betaine, der Aminoxide bzw. entsprechender Derivate davon.

21. Verbindung nach mindestens einem der Ansprüche 14 bis 20,
**dadurch gekennzeichnet, dass** n steht für eine Zahl aus dem Bereich 8 bis 24.

22. Verfahren zur Herstellung einer Verbindung nach Formel Ia, Ib oder Ic, **dadurch gekennzeichnet, dass** zuerst eine Verbindung der Formel IV
Y-Spacer-OH IV
hergestellt wird und diese dann, soweit X in der Verbindung der Formel Ia, Ib oder Ic verschieden von OH ist, durch Modifikation der OH-Gruppe in an sich bekannter Weise zur Verbindung der Formel Ia, Ib oder Ic umgesetzt wird.

23. Verfahren zur Herstellung einer Verbindung nach Formel IIa,
**dadurch gekennzeichnet, dass** zuerst eine Verbindung der Formel V
F₃CO-(CH₂)ₙ-OH V
durch Umsetzung eines geschützten Diols zum geschützten Monotrifluormethoxy-alkohol und anschließendes Entschützen hergestellt wird und diese dann, soweit X in der Verbindung der Formel IIa verschieden von OH ist, durch Modifikation der OH-Gruppe in an sich bekannter Weise zur Verbindung der Formel IIa umgesetzt wird.

24. Verfahren zur Herstellung einer Verbindung nach Formel IIIa, IIIb bzw. IIIc, **dadurch gekennzeichnet, dass** zuerst eine Verbindung der Formel IIIb, in der X steht für OH hergestellt wird und diese, wenn eine Verbindung der Formel IIIa bzw. IIIc hergestellt werden soll, durch Halogenwasserstoff-Eliminierung und, wenn eine Verbindung der Formel IIIa hergestellt werden soll, anschließende Hydrierung umgesetzt wird und anschließend, soweit X in der Verbindung nach Formel IIIa, IIIb bzw. IIIc verschieden von OH ist, das Produkt durch Modifikation der OH-Gruppe in an sich bekannter Weise zur Verbindung nach Formel IIIa, IIIb bzw. IIIc umgesetzt wird.

25. Mittel enthaltend mindestens eine oberflächenaktive Verbindung mit mindestens einer Endgruppe Y, wobei Y steht für CF₃O- oder F₅S- und wobei Verbindungen verwendet werden, die der Formel Ia, Ib oder Ic
Y-(CH₂)ₙ-X Ia
Y-CH₂-CH(Hal)-(CH₂)₍ₙ₋₁₎-X Ib
Y-CH=CH-(CH₂)₍ₙ₋₁₎-X Ic
worin Y steht für CF₃O- oder F₅S-,
n steht für eine ganze Zahl aus dem Bereich 4 bis 28 und
X steht für eine kationische, nichtionische, ampothere oder anionische polare Gruppe oder eine polymerisierbare Gruppe,
(Hal) steht für F, Cl, Br oder I,
sowie entsprechender Salze der Verbindungen nach Formel Ia, Ib bzw. Ic
entsprechen, und einen für den jeweiligen Verwendungszweck geeigneten Träger sowie ggf. weitere spezifische Aktivstoffe.

26. Mittel nach Anspruch 25, **dadurch gekennzeichnet, dass** es sich bei dem Mittel um Farb- und Lackzubereitungen, Feuerlöschmittel, Schmierstoffe, Wasch- und Reinigungsmittel, Enteiser oder Hydrophobiermittel zur Textilausrüstung oder Glasbehandlung handelt.

## Claims

1. Use of compounds containing at least one hydrophobic end group Y, where Y stands for CF₃O- or F₅S-, the end group Y is bonded to a saturated or unsaturated, branched or unbranched hydrocarbon unit and compounds are used which conform to the formula Ia, Ib or Ic
Y-(CH₂)ₙ-X Ia
Y-CH₂-CH(Hal)-(CH₂)₍ₙ₋₁₎-X Ib
Y-CH=CH-(CH₂)₍ₙ₋₁₎-X Ic
in which Y stands for CF₃O- or F₅S-,
n stands for an integer from the range 4 to 28 and
X stands for a cationic, nonionic, amphoteric or anionic polar group or a polymerisable group,
(Hal) stands for F, Cl, Br or I,
and corresponding salts of the compounds of the formula Ia, Ib or Ic, as surface-active agents.

2. Use according to Claim 1, **characterised in that** the surface-active compound is used as surfactant.

3. Use according to Claim 1, **characterised in that** the surface-active compound is used as hydrophobicising agent.

4. Use according to Claim 1, **characterised in that** the surface-active compound is used as interface promoter.

5. Use according to Claim 1, **characterised in that** the surface-active compound is used as viscosity reducer, in particular in paints, coatings or compositions for surface coating.

6. Use according to Claim 1, **characterised in that** the surface-active compound is used as foam stabiliser, in particular in fire-extinguishing foams.

7. Use according to Claim 1, **characterised in that** the surface-active compound is used as emulsifier, in particular for the preparation of fluoropolymers.

8. Use according to Claim 1, **characterised in that** the surface-active compound is used as antimicrobial active ingredient, in particular as reagent for antimicrobial surface modification.

9. Use according to at least one of the preceding claims, **characterised in that** X stands for an anionic polar group selected from -COOM, -SO₃M, -OSO₃M, -PO₃M₂, -OPO₃M₂, -(OCH₂CH₂)ₘ-O-(CH₂)ₒ-COOM, -(OCH₂CH₂)ₘ-O-(CH₂)ₒ-SO₃M, -(OCH₂CH₂)ₘ-O-(CH₂)ₒ-OSO₃M, -(OCH₂CH₂)ₘ-O-(CH₂)ₒ-PO₃M₂, -(OCH₂CH₂)ₘ-O-(CH₂)ₒ-OPO₃M₂,
where M stands for H or an alkali metal ion, preferably Li⁺, Na⁺ or K⁺, or NH₄⁺,
m stands for an integer from the range 1 to 1000 and
o stands for an integer selected from 1, 2, 3 or 4.

10. Use according to at least one of Claims 1 to 9, **characterised in that** X stands for a cationic polar group selected from -NR¹R²R³⁺ Z⁻, -PR¹R²R³⁺ Z⁻, 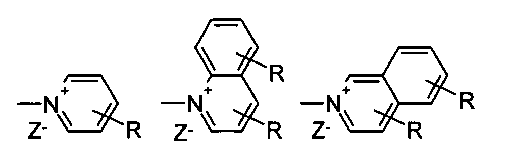 where R stands for H or C₁₋₄-alkyl in any desired position,
Z⁻ stands for Cl⁻, Br⁻, I⁻, CH₃SO₃⁻, CF₃SO₃⁻, CH₃PhSO₃⁻, PhSO₃⁻,
R¹, R² and R³ each, independently of one another, stand for H, C₁₋₃₀-alkyl, Ar or -CH₂Ar and
Ar stands for an unsubstituted or mono- or polysubstituted aromatic ring or fused ring system having 6 to 18 C atoms, in which, in addition, one or two CH groups may be replaced by N.

11. Use according to at least one of Claims 1 to 9, **characterised in that** X stands for a nonionic polar group selected from -Cl, -Br, -I, -(OCH₂CH₂)ₘ-OH, -O-(glycoside)ₒ, -(OCH₂CH₂)ₘ-OCH₂-CHOH-CH₂-OH, -(OCH₂CH₂)ₘ-OCH₂Ar(-NCO)ₚ, -(OCH₂CH₂)ₘ-OAr(-NCO)ₚ, -SiR¹R²Z, -SiR¹Z₂, -SiZ₃, -R²-COZ, -(OCH₂CH₂)ₘ-SO₂CH=CH₂, -SO₂Z, 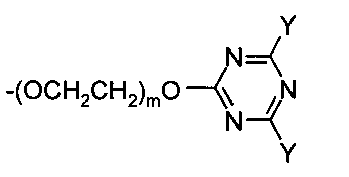 m stands for an integer from the range 0 to 1000,
n stands for 0 or 1 and
o stands for an integer from the range 1 to 10,
p stands for 1 or 2,
R¹ and R² each, independently of one another, stand for C₁₋₃₀-alkyl,
Ar or -CH₂Ar and
Ar stands for an unsubstituted or mono- or polysubstituted aromatic ring or fused ring system having 6 to 18 C atoms, in which, in addition, one or two CH groups may be replaced by N and
glycoside stands for an etherified carbohydrate, preferably for a mono-, di-, tri- or oligoglucoside,
all Z each, independently of one another, stand for -H, -Cl, -F, -NR¹R², -OR¹, -N-imidazolyl and
Y stands for Cl or F.

12. Use according to at least one of Claims 1 to 9, **characterised in that** X stands for a polymerisable group selected from -(OCH₂CH₂)ₘOCOCR-CH₂, -(OCH₂CH₂)ₘ-OCR=CH₂, 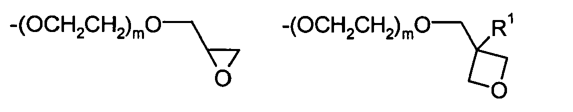 where m stands for an integer from the range 0 to 1000 and R and R¹ stand for H or C₁₋₄-alkyl.

13. Use according to at least one of Claims 1 to 9, **characterised in that** X stands for an amphoteric group selected from the functional groups of the acetyldiamines, the N-alkylamino acids, the betaines, the amine oxides or corresponding derivatives thereof.

14. Compound of the formula IIa, IIb or IIc
CF₃O-(CH₂)ₙ-X IIa
CF₃O-CH₂-CH(Hal)-(CH₂)(ₙ₋₁₎-X IIb
CF₃O-CH=CH-(CH₂)₍ₙ₋₁₎-X IIc
in which
n stands for an integer from the range 4 to 28 and
X stands for a cationic, nonionic, amphoteric or anionic polar group or a polymerisable group,
(Hal) stands for F, Cl, Br or I,
and corresponding salts of the compounds of the formula IIa, IIb or IIc, where the compound CF₃-O-CH₂-COOH is excluded.

15. Compound of the formula IIIa, IIIb or IIIc
F₅S-(CH₂)ₙ-X IIIa
F₅S-CH₂-CH(Hal)-(CH₂)(ₙ₋₁)-X IIIb
F₅S-CH=CH-(CH₂)₍ₙ₋₁₎-X IIIc
in which
n stands for an integer from the range 4 to 28 and
X stands for a cationic, nonionic, amphoteric or anionic polar group or a polymerisable group,
(Hal) stands for F, Cl, Br or I,
and corresponding salts of the compounds of the formula IIIa, IIIb or IIIc, where the compounds F₅S-CH₂-CHCl-(CH₂)₈-X¹, where X¹ stands for OH, OC(=O)CH₃, Br, C(=O)OC₂H₅, C(=O)CH₃, are excluded.

16. Compound according to at least one of Claims 14 or 15, **characterised in that** X stands for an anionic polar group selected from -COOM, -SO₃M, -OSO₃M, -PO₃M₂, -OPO₃M₂, -(OCH₂CH₂)ₘ-O-(CH₂)ₒ-COOM, -(OCH₂CH₂)ₘ-O-(CH₂)ₒ-SO₃M, -(OCH₂CH₂)ₘ-O-(CH₂)ₒ-OSO₃M, -(OCH₂CH₂)ₘ-O-(CH₂)ₒ-PO₃M₂, -(OCH₂CH₂)ₘ-O-(CH₂)ₒ-OPO₃M₂,
where M stands for H or an alkali metal ion, preferably Li⁺, Na⁺ or K⁺, or NH₄⁺,
m stands for an integer from the range 1 to 1000 and
o stands for an integer selected from 1, 2, 3 or 4.

17. Compound according to at least one of Claims 14 or 15, **characterised in that** X stands for a cationic polar group selected from -NR¹R²R³⁺ Z⁻, -PR¹R²R³⁺ + Z⁻, 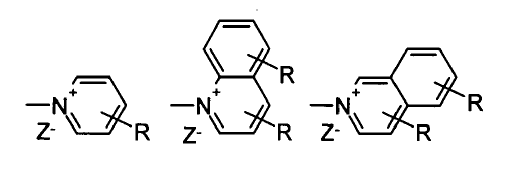 where R stands for H or C₁₋₄-alkyl in any desired position,
Z⁻ stands for Cl⁻, Br⁻, I⁻, CH₃SO₃⁻, CF₃SO₃⁻, CH₃PhSO₃⁻, PhSO₃⁻,
R¹, R² and R³ each, independently of one another, stand for H, C₁₋₃₀-alkyl, Ar or -CH₂Ar and
Ar stands for an unsubstituted or mono- or polysubstituted aromatic ring or fused ring system having 6 to 18 C atoms, in which, in addition, one or two CH groups may be replaced by N.

18. Compound according to at least one of Claims 14 or 15, **characterised in that** X stands for a nonionic polar group selected from -Cl, -Br, -I, -(OCH₂CH₂)ₘ-OH, -O-(glycoside)ₒ, -(OCH₂CH₂)ₘ-OCH₂-CHOH-CH₂-OH, -(OCH₂CH₂)ₘ-OCH₂Ar(-NCO)ₚ, -(OCH₂CH₂)ₘ-OAr(-NCO)ₚ, -SiR¹R²Z, -SiR¹Z₂, -SiZ₃, -COZ, -(OCH₂CH₂)ₘ-SO₂CH=CH₂, -SO₂Z, 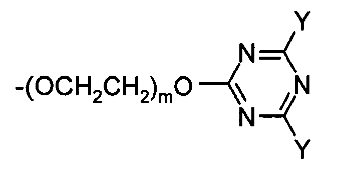 m stands for an integer from the range 0 to 1000,
n stands for 0 or 1 and
o stands for an integer from the range 1 to 10,
p stands for 1 or 2,
R¹ and R² each, independently of one another, stand for C₁₋₃₀-alkyl, Ar or -CH₂Ar and
Ar stands for an unsubstituted or mono- or polysubstituted aromatic ring or fused ring system having 6 to 18 C atoms, in which, in addition, one or two CH groups may be replaced by N, and
glycoside stands for an etherified carbohydrate, preferably for a mono-, di-, tri- or oligoglucoside,
all Z each, independently of one another, stand for -H, -Cl, -F, -NR¹R², -OR¹, -N-imidazolyl and
Y stands for Cl or F.

19. Compound according to at least one of Claims 14 or 15, **characterised in that** X stands for a polymerisable group selected from -(OCH₂CH₂)ₘOCOCR=CH₂, -(OCH₂CH₂)ₘ-OCR=CH₂, 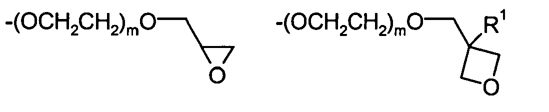 where m stands for an integer from the range 0 to 1000 and R and R¹ stand for H or C₁₋₄-alkyl.

20. Compound according to at least one of Claims 14 or 15, **characterised in that** X stands for an amphoteric group selected from the functional groups of the acetyldiamines, the N-alkylamino acids, the betaines, the amine oxides or corresponding derivatives thereof.

21. Compound according to at least one of Claims 14 to 20, **characterised in that** n stands for a number from the range 8 to 24.

22. Process for the preparation of a compound of the formula Ia, Ib or Ic, **characterised in that** firstly a compound of the formula IV
Y-spacer-OH IV
is prepared and then, if X in the compound of the formula Ia, Ib or Ic is other than OH, is converted into the compound of the formula la, Ib or Ic by modification of the OH group in a manner known per se.

23. Process for the preparation of a compound of the formula IIa, **characterised in that** firstly a compound of the formula V
F₃CO-(CH₂)ₙ-OH V
is prepared by conversion of a protected diol into the protected monotrifluoromethoxy alcohol followed by deprotection, and then, if X in the compound of the formula IIa is other than OH, is converted into the compound of the formula IIa by modification of the OH group in a manner known per se.

24. Process for the preparation of a compound of the formula IIIa, IIIb or IIIc, **characterised in that** firstly a compound of the formula IIIb in which X stands for OH is prepared and, if a compound of the formula IIIa or IIIc is to be prepared, is reacted by elimination of hydrogen halide and, if a compound of the formula IIIa is to be prepared, subsequent hydrogenation, and subsequently, if X in the compound of the formula IIIa, IIIb or IIIc is other than OH, the product is converted into the compound of the formula IIIa, IIIb or IIIc by modification of the OH group in a manner known per se.

25. Composition comprising at least one surface-active compound containing at least one end group Y, where Y stands for CF₃O- or F₅Sand where compounds are used which conform to the formula Ia, Ib or Ic
Y-(CH₂)ₙ-X Ia
Y-CH₂-CH(Hal)-(CH₂)₍ₙ₋₁₎-X Ib
Y-CH=CH-(CH₂)₍ₙ₋₁₎-X Ic
in which Y stands for CF₃O- or F₅S-,
n stands for an integer from the range 4 to 28 and
X stands for a cationic, nonionic, amphoteric or anionic polar group or a polymerisable group,
(Hal) stands for F, Cl, Br or I,
and corresponding salts of the compounds of the formula Ia, Ib or Ic,
and a carrier which is suitable for the particular application and optionally further specific active ingredients.

26. Composition according to Claim 25, **characterised in that** the composition is a paint or coating composition, fire-extinguishing composition, lubricant, washing or cleaning composition, de-icer or hydrophobicising agent for textile finishing or glass treatment.

## Revendications

1. Utilisation de composés contenant au moins un groupement terminal hydrophobe Y, où Y représente CF₃O- ou F₅S-, le groupement terminal Y étant lié à une unité hydrocarbonée ramifiée ou non ramifiée, saturée ou insaturée, et on utilise des composés répondant à la formule Ia, Ib ou Ic
Y-(CH₂)ₙ-X Ia
Y-CH₂-CH(Hal)-(CH₂)₍ₙ₋₁₎-X Ib
Y-CH=CH-(CH₂)₍ₙ₋₁₎-X Ic
dans laquelle Y représente CF₃O- ou F₅S-,
n représente un nombre entier dans la plage allant de 4 à 28 et
X représente un groupement polaire cationique, non ionique, amphotère ou anionique ou un groupement polymérisable,
(Hal) représente F, Cl, Br ou I,
et les sels correspondants des composés de formule Ia, Ib ou Ic, comme agents tensioactifs.

2. Utilisation selon la revendication 1, **caractérisée en ce que** le composé tensioactif est utilisé comme tenside.

3. Utilisation selon la revendication 1, **caractérisée en ce que** le composé tensioactif est utilisé comme agent hydrophobisant.

4. Utilisation selon la revendication 1, **caractérisée en ce que** le composé tensioactif est utilisé comme promoteur d'interface.

5. Utilisation selon la revendication 1, **caractérisée en ce que** le composé tensioactif est utilisé comme réducteur de viscosité, en particulier dans les peintures, les revêtements ou les compositions pour revêtement de surface.

6. Utilisation selon la revendication 1, **caractérisée en ce que** le composé tensioactif est utilisé comme stabilisateur de mousse, en particulier dans les mousses extinctrices.

7. Utilisation selon la revendication 1, **caractérisée en ce que** le composé tensioactif est utilisé comme émulsifiant, en particulier pour la préparation de polymères fluorés.

8. Utilisation selon la revendication 1, **caractérisée en ce que** le composé tensioactif est utilisé comme ingrédient actif antimicrobien, en particulier comme réactif pour une modification de surface anti-microbienne.

9. Utilisation selon au moins l'une des revendications précédentes, **caractérisée en ce que** X représente un groupement polaire anionique choisi parmi -COOM, -SO₃M, -OSO₃M, -PO₃M₂, -OPO₃M₂, -(OCH₂CH₂)ₘ-O-(CH₂)ₒ-COOM, -(OCH₂CH₂)ₘ-O-(CH₂)ₒ-SO₃M, -(OCH₂CH₂)ₘ-O-(CH₂)ₒ-OS0₃M, -(OCH₂CH₂)ₘ-O-(CH₂)ₒ-PO₃M₂, -(OCH₂CH₂)ₘ-O-(CH₂)ₒ-OPO₃M₂,
où M représente H ou un ion de métal alcalin, préférablement Li⁺, Na⁺ ou K⁺, ou NH₄⁺,
m représente un nombre entier dans la plage allant de 1 à 1000 et
o représente un nombre entier choisi parmi 1, 2, 3 ou 4.

10. Utilisation selon au moins l'une des revendications 1 à 9, **caractérisée en ce que** X représente un groupement polaire cationique choisi parmi -NR¹R²R³⁺ Z⁻, -PR¹R²R³⁺ Z⁻, 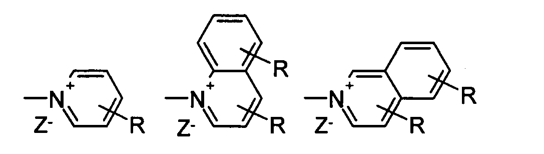 où R représente H ou C₁₋₄-alkyle dans une position désirée quelconque,
Z- représente Cl⁻, Br⁻, I⁻, CH₃SO₃⁻, CF₃SO₃⁻, CH₃PhSO₃⁻, PhSO₃⁻,
R¹, R² et R³ représentent chacun, indépendamment l'un de l'autre, H, C₁₋₃₀-alkyle, Ar ou -CH₂Ar et
Ar représente un système cyclique condensé ou cycle aromatique non substitué ou mono- ou polysubstitué ayant de 6 à 18 atomes de C, où, en outre, un ou deux groupements CH peuvent être remplacés par N.

11. Utilisation selon au moins l'une des revendications 1 à 9, **caractérisée en ce que** X représente un groupement polaire non ionique choisi parmi -Cl, -Br, -I, -(OCH₂CH₂)ₘ-OH, -O-(glycoside)ₒ, -(OCH₂CH₂)ₘ-OCH₂-CHOH-CH₂-OH, -(OCH₂CH₂)ₘ-OCH₂Ar(-NCO)ₚ, -(OCH₂CH₂)ₘ-OAr(-NCO)ₚ, -SiR¹R²Z, -SiR¹Z₂, -SiZ₃, -R²-COZ, -(OCH₂CH₂)ₘ-SO₂CH=CH₂, -SO₂Z, 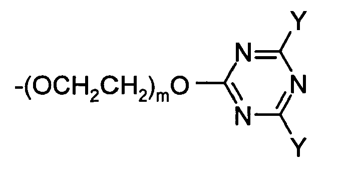 m représente un nombre entier dans la plage allant de 0 à 1000,
n représente 0 ou 1 et
o représente un nombre entier dans la plage allant de 1 à 10,
p représente 1 ou 2,
R¹ et R² représentent chacun, indépendamment l'un de l'autre, C₁₋₃₀-alkyle, Ar ou -CH₂Ar et
Ar représente un système cyclique condensé ou cycle aromatique non substitué ou mono- ou polysubstitué ayant de 6 à 18 atomes de C, où, en outre, un ou deux groupements CH peuvent être remplacés par N et
glycoside représente un glucide éthérifié, préférablement un mono-, di-, tri- ou oligoglucoside,
tous les Z représentent chacun, indépendamment l'un de l'autre, -H, -CI, -F, -NR¹R², -OR¹, -N-imidazolyle et
Y représente Cl ou F.

12. Utilisation selon au moins l'une des revendications 1 à 9, **caractérisée en ce que** X représente un groupement polymérisable choisi parmi -(OCH₂CH₂)ₘOCOCR=CH₂, -(OCH₂CH₂)ₘ-OCR=CH₂, 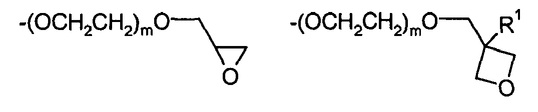 où m représente un nombre entier dans la plage allant de 0 à 1000 et R et R¹ représentent H ou C₁₋₄-alkyle.

13. Utilisation selon au moins l'une des revendications 1 à 9, **caractérisée en ce que** X représente un groupement amphotère choisi parmi les groupements fonctionnels des acétyldiamines, des acides N-alkylaminés, des bétaïnes, des oxydes d'amine ou des dérivés correspondants de ceux-ci.

14. Composé de formule IIa, IIb ou IIc
CF₃O-(CH₂)ₙ-X IIa
CF₃O-CH₂-CH(Hal)-(CH₂)₍ₙ₋₁₎-X IIb
CF₃O-CH=CH-(CH₂)₍ₙ₋₁₎-X IIc
dans laquelle
n représente un nombre entier dans la plage allant de 4 à 28 et
X représente un groupement polaire cationique, non ionique, amphotère ou anionique ou un groupement polymérisable,
(Hal) représente F, CI, Br ou I,
et les sels correspondants des composés de formule IIa, IIb ou IIc,
où le composé CF₃-O-CH₂-COOH est exclu.

15. Composé de formule IIIa, IIIb ou IIIc
F₅S-(CH₂)ₙ-X IIIa
F₅S-CH₂-CH(Hal)-(CH₂)₍ₙ₋₁₎-X IIIb
F₅S-CH=CH-(CH₂)₍ₙ₋₁₎-X IIIc
dans laquelle
n représente un nombre entier dans la plage allant de 4 à 28 et
X représente un groupement polaire cationique, non ionique, amphotère ou anionique ou un groupement polymérisable,
(Hal) représente F, Cl, Br ou I,
et les sels correspondants des composés de formule IIIa, IIIb ou IIIc,
où les composés F₅S-CH₂-CHCl-(CH₂)₈-X¹, où X¹ représente OH, OC(=O)CH₃, Br, C(=O)OC₂H₅, C(=O)CH₃, sont exclus.

16. Composé selon au moins l'une des revendications 14 ou 15, **caractérisé en ce que** X représente un groupement polaire anionique choisi parmi -COOM, -SO₃M, -OSO₃M, -PO₃M₂, -OPO₃M₂, -(OCH₂CH₂)ₘ-O-(CH₂)ₒ-COOM, -(OCH₂CH₂)ₘ-O-(CH₂)ₒ-SO₃M, -(OCH₂CH₂)ₘ-O-(CH₂)ₒ-OSO₃M, -(OCH₂CH₂)ₘ-O-(CH₂)ₒ-PO₃M₂, -(OCH₂CH₂)ₘ-O-(CH₂)ₒ-OPO₃M₂,
où M représente H ou un ion de métal alcalin, préférablement Li⁺, Na⁺ ou K⁺, ou NH₄⁺,
m représente un nombre entier dans la plage allant de 1 à 1000 et
o représente un nombre entier choisi parmi 1, 2, 3 ou 4.

17. Composé selon au moins l'une des revendications 14 ou 15, **caractérisé en ce que** X représente un groupement polaire cationique choisi parmi -NR¹R²R³⁺ Z⁻, -PR¹R²R³⁺ Z⁻, 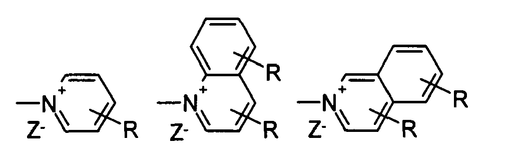 où R représente H ou C₁₋₄-alkyle dans une position désirée quelconque,
Z⁻ représente Cl⁻, Br⁻, I⁻, CH₃SO₃⁻, CF₃SO₃⁻, CH₃PhSO₃⁻, PhSO₃⁻,
R¹, R² et R³ représentent chacun, indépendamment l'un de l'autre, H, C₁₋₃₀-alkyle, Ar ou -CH₂Ar et
Ar représente un système cyclique condensé ou cycle aromatique non substitué ou mono- ou polysubstitué ayant de 6 à 18 atomes de C, où, en outre, un ou deux groupements CH peuvent être remplacés par N.

18. Composé selon au moins l'une des revendications 14 ou 15, **caractérisé en ce que** X représente un groupement polaire non ionique choisi parmi -CI, -Br, -I, -(OCH₂CH₂)ₘ-OH, -O-(glycoside)ₒ, -(OCH₂CH₂)ₘ-OCH₂-CHOH-CH₂-OH, -(OCH₂CH₂)ₘ-OCH₂Ar(-NCO)ₚ, -(OCH₂CH₂)ₘ-OAr(-NCO)ₚ, -SiR¹R²Z, -SiR¹Z₂, -SiZ₃, -COZ, -(OCH₂CH₂)ₘ-SO₂CH=CH₂, -SO₂Z, 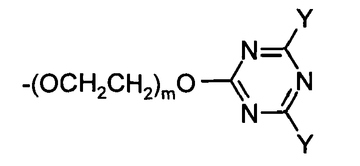 m représente un nombre entier dans la plage allant de 0 à 1000,
n représente 0 ou 1 et
o représente un nombre entier dans la plage allant de 1 à 10,
p représente 1 ou 2,
R¹ et R² représentent chacun, indépendamment l'un de l'autre, C₁₋₃₀-alkyle, Ar ou -CH₂Ar et
Ar représente un système cyclique condensé ou cycle aromatique non substitué ou mono- ou polysubstitué ayant de 6 à 18 atomes de C, où, en outre, un ou deux groupements CH peuvent être remplacés par N, et
glycoside représente un glucide éthérifié, préférablement un mono-, di-, tri- ou oligoglucoside,
tous les Z représentent chacun, indépendamment l'un de l'autre, -H, -Cl, -F, -NR¹R², -OR¹, -N-imidazolyle et
Y représente Cl ou F.

19. Composé selon au moins l'une des revendications 14 ou 15, **caractérisé en ce que** X représente un groupement polymérisable choisi parmi -(OCH₂CH₂)ₘOCOCR=CH₂, -(OCH₂CH₂)ₘ-OCR=CH₂, 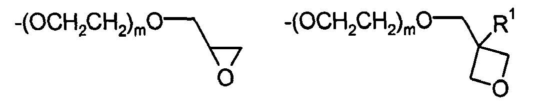 où m représente un nombre entier dans la plage allant de 0 à 1000 et R et R¹ représentent H ou C₁₋₄-alkyle.

20. Composé selon au moins l'une des revendications 14 ou 15, **caractérisé en ce que** X représente un groupement amphotère choisi parmi les groupements fonctionnels des acétyldiamines, des acides N-alkylaminés, des bétaïnes, des oxydes d'amine ou des dérivés correspondants de ceux-ci.

21. Composé selon au moins l'une des revendications 14 à 20, **caractérisé en ce que** n représente un nombre dans la plage allant de 8 à 24.

22. Procédé de préparation d'un composé de formule Ia, Ib ou Ic, **caractérisé en ce que** tout d'abord un composé de formule IV
Y-espaceur-OH IV
est préparé puis, si X dans le composé de formule Ia, Ib ou Ic est autre que OH, est converti en composé de formule Ia, Ib ou Ic par modification du groupement OH d'une manière connue en soi.

23. Procédé de préparation d'un composé de formule IIa, **caractérisé en ce que** tout d'abord un composé de formule V
F₃CO-(CH₂)ₙ-OH V
est préparé par conversion d'un diol protégé en monotrifluorométhoxy alcool protégé suivie d'une déprotection, puis, si X dans le composé de formule IIa est autre que OH, est converti en composé de formule IIa par modification du groupement OH d'une manière connue en soi.

24. Procédé de préparation d'un composé de formule IIIa, IIIb ou IIIc, **caractérisé en ce que** tout d'abord un composé de formule IIIb dans laquelle X représente OH est préparé et, si un composé de formule IIIa ou IIIc doit être préparé, est réagi par élimination d'halogénure d'hydrogène et, si un composé de formule IIIa doit être préparé, une hydrogénation ultérieure, puis, si X dans le composé de formule IIIa, IIIb ou IIIc est autre que OH, le produit est converti en composé de formule IIIa, IIIb ou IIIc par modification du groupement OH d'une manière connue en soi.

25. Composition comprenant au moins un composé tensioactif contenant au moins un groupement terminal Y, où Y représente CF₃O- ou F₅S- et où on utilise des composés répondant à la formule Ia, Ib ou Ic
Y-(CH₂)ₙ-X Ia
Y-CH₂-CH(Hal)-(CH₂)₍ₙ₋₁₎-X Ib
Y-CH=CH-(CH₂)₍ₙ₋₁₎-X Ic
dans laquelle Y représente CF₃O- ou F₅S-,
n représente un nombre entier dans la plage allant de 4 à 28 et
X représente un groupement polaire cationique, non ionique, amphotère ou anionique ou un groupement polymérisable,
(Hal) représente F, CI, Br ou I,
et les sels correspondants des composés de formule Ia, Ib ou Ic,
et un véhicule qui est convenable pour l'application particulière et éventuellement d'autres ingrédients actifs spécifiques.

26. Composition selon la revendication 25, **caractérisée en ce que** la composition est une composition de revêtement ou de peinture, une composition extinctrice, une composition lubrifiante, lavante ou nettoyante, un agent hydrophobisant ou dégivrant pour le finissage des textiles ou le traitement du verre.
